(19)

Europäisches
Patentamt
European
Patent Office
Office européen
des brevets

(11) **EP 3 725 895 A1**

(12) **EUROPEAN PATENT APPLICATION**

(43) Date of publication:
   **21.10.2020   Bulletin 2020/43**

(51) Int Cl.:
   ***C12Q 1/6858*** *(2018.01)*

(21) Application number: **19170245.5**

(22) Date of filing: **18.04.2019**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**
   Designated Validation States:
   **KH MA MD TN**

(71) Applicant: **Helm, Mark**
   **55126 Mainz (DE)**

(72) Inventors:
   • **HELM, Mark**
     **55126 Mainz (DE)**
   • **PICHOT, Florian**
     **55128 Mainz (DE)**
   • **WERNER, Stephan**
     **55126 Mainz (DE)**

(74) Representative: **Müller-Boré & Partner**
   **Patentanwälte PartG mbB**
   **Friedenheimer Brücke 21**
   **80639 München (DE)**

(54)   **DETECTION OF NUCLEIC ACID MODIFICATIONS BY USING CHEMICAL DEAMINATION**

(57)   The present invention relates to methods for detecting the position of a nucleotide modification, selected from the group consisting of $N^6$-methyladenosine (m6A) and N4-methylcytidine (m4C), in one or more RNA species. Said methods are based on a deamination of the RNA followed by reverse transcription and sequencing of the resulting cDNAs.

EP 3 725 895 A1

**Description**

The present invention relates to methods for detecting the position of a nucleotide modification, selected from the group consisting of $N^6$-methyladenosine (m6A) and $N^4$-methylcytidine (m4C), in one or more RNA species. Said methods are based on a deamination of the RNA followed by reverse transcription and sequencing of the resulting cDNAs.

Interest in the field of RNA modification research has grown considerably in recent years. Over 160 types of post-transcriptional RNA modifications have been identified, a majority of which had been known for decades. However, advances in technology have only recently made it possible to investigate these modifications more precisely through sufficiently sensitive tools and high-resolution techniques.

In order to understand in which areas of eukaryotic biological processes these modifications play a role, it is of great interest to determine their exact occurrence in RNA species, ideally with single-nucleotide resolution. For this purpose, technologies such as Next-Generation-Sequencing (NGS) are applied, enabling the precise position of modified sites in RNA sequences. The identification and characterization of diverse types of RNA base modifications in several RNAs have shaped the emerging field of "epitranscriptomics". The transcriptome-wide analysis of RNA modifications already facilitated the detection of several modifications, including for instance 5-methylcytidine (m5C) and pseudouridine (ψ). Especially the identification and quantification of modifications in low abundant RNA species such as mRNA encounters great interest and will further advance this rapidly growing research area. The importance of some modifications in affecting normal development and disease has recently been shown in relation to their influence on control of turnover and/or translation of transcripts during cell-state transitions. In particular, the $N^6$-methyladenosine (m6A) modification is a key regulator of mammalian gene expression.

$N^6$-methyladenosine is one of the most-abundant and well-studied mRNA modifications and is also present in long non-coding RNAs (lncRNAs) and primary microRNAs (pri-miRNAs). In mammalian cells, up to 40% of all different mRNA species may contain m6A and furthermore, methylated mRNAs may contain multiple m6A sites per transcript. Its regulatory biological function is controlled by writer, reader and eraser proteins. Functional studies strongly suggest that m6A methylation serves as a fundamental mechanism in synchronizing groups of transcripts for coordinated metabolism, translation and decay. Further, m6A plays an essential role during development by influencing embryonic and adult stem cell differentiation through timely and coordinated protein synthesis as well as transcriptome switching. Misregulation of these processes can lead to embryonic lethality and human diseases, including for example type 2 diabetes, autism, schizophrenia, Alzheimer's disease, major depressive disorder, and various types of cancer.

Currently, the main research focus is on the role of m6A and its regulatory enzymes in cancer. The contribution of mRNA modifications to the survival and growth of tumor cells could be proven. Links between m6A writer and eraser proteins to acute myeloid leukemia (AML), endometrial cancer and glioblastoma were recently discovered. Despite these studies impressively demonstrating the roles of m6A in various processes, like cancer cells hijacking mRNA m6A methylation to upregulate translation, the understanding remains incomplete.

Some recent research already demonstrated transcriptome-wide detection approaches for m6A. In 2012, two groups published a method independently of each other, both working with antibody-based enrichment methodologies for targeted m6A detection. However, both methods lacked precise single-nucleotide resolution mapping, and only limited sequence areas in which modified nucleotides were present could be determined. A second negative aspect of these methods concerned the specificity of the commercial antibodies used in these studies, which were not able to provide distinguishability between m6A versus $N^6$,2'-O-dimethyladenosine (m6Am). To address these obvious limitations, the existing methods were extended to include ultraviolet light-induced antibody-RNA crosslinking and reverse transcription. However, as soon as the data is noisy, it generates significant amounts of false negatives and false positives. As a result, in this case the forecasting power for modification sites is also subject to tight limits. Further, the limitations regarding antibodies lacking specificity have not been fixed either. Furthermore, the known methods require large amounts of RNA and expensive materials for enrichment. With regard to routine application in an economic way, the premise must certainly be to minimize efforts and costs.

Other known approaches serve as validation tools for single m6A sites but cannot be utilized for large-scale m6A detection. In this respect, the site-specific cleavage and radioactive-labeling followed by ligation-assisted extraction and thin-layer chromatography (SCARLET) method known in the art, to determine the m6A status is a promising method. Unfortunately, this approach is very laborious, and samples can only be processed in a very small dimensional range, which makes it very time-consuming and expensive as a consequence. Another idea to detect m6A at single-nucleotide resolution was established, taking advantage of the possibility to determine modified sites in sequencing data by exploiting the generation of characteristic error patterns which are introduced at modified sites during reverse transcription of RNA into cDNA. The analysis of generated patterns in sequencing data, the so-called RT signature, could be successfully applied for specific modification detection of $N^1$-methyladenosine (m1A). This method requires changes at the Watson-Crick edge or specific mutation of the enzyme's active site to affect base-pairing during reverse transcription and therefore cannot be applied to every modification one-to-one. In this context, a polymerase with reverse transcriptase activity was engineered to create errors by incorporating wrong nucleotides at generally reverse transcription silent m6A sites during

reverse transcription of RNA templates to cDNA. The engineered polymerase was applicable for limited sequences, but the low processing and the error-prone behavior towards other modified sites did not allow its application in large-scale transcriptome studies. Recently, additional techniques for m6A verification were suggested, all of which lack the application to transcriptomic m6A sites and some of which also lack sequencing application in general. All in all, these methods are still at the beginning of development, so their applicability can therefore be predicted only with difficulty or not at all. In addition, at the present time and stage of development, no reliable statements can be made about their accuracy and susceptibility to errors.

In summary, although it is currently possible to map m6A transcriptome-wide, the desired single-nucleotide resolution is lacking. Further, a quantification, *i.e.*, the determination of the degree of methylation at a particular sequence in molar percentage, is missing. Furthermore, there are methods that can provide either quantification or single-nucleotide resolution at certain sites, but cannot be applied in a transcriptome-wide manner.

New methods to determine the transcriptome-wide distribution of RNA modifications at single-nucleotide resolution would help to further investigate their impact on the modulation of RNA processing or protein translation machineries.

Therefore, the technical problem underlying the present invention is the provision of methods for the detection of RNA nucleotide modifications with single-nucleotide resolution in extensive RNA samples, e.g. in a transcriptome-wide manner.

The solution to the above-mentioned technical problem is achieved by the embodiments characterized in the claims.

In particular, the present invention relates to a method for detecting the position of a nucleotide modification, selected from the group consisting of $N^6$-methyladenosine (m6A) and $N^4$-methylcytidine (m4C) in one or more RNA species, comprising the steps:

    (a) providing a sample comprising said one or more RNA species,
    (b) subjecting said sample to a deamination reaction to obtain deaminated RNAs,
    (c) subjecting said deaminated RNAs to reverse transcription using a reverse transcriptase (RT) to obtain respective cDNAs,
    (d) sequencing said cDNAs, and
    (e) determining the presence of m6A at a given position in the RNA, in case the sequencing data show an adenosine (A) in the corresponding position, or the presence of m4C at a given position in the RNA, in case the sequencing data show a cytidine (C) in the corresponding position.

As used herein, the term "detecting the position of a nucleotide modification, selected from the group consisting of m6A and m4C in one or more RNA species" relates to the determination of the presence or absence and the location of one of said modifications for every nucleoside in an RNA sequence. In particular, the methods of the present invention do not require any predetermination of a specific RNA sequence or a specific location in a given sequence. Instead, said methods allow the analysis of any given RNA-containing sample. In case any RNA sequence in said sample contains an m6A or m4C, the presence and exact location of said m6A or m4C in said sequence can be determined.

As used herein, the term "sample containing one or more RNA species" relates to any sample containing, or being suspected of containing, any one or more RNAs. In specific embodiments, said one or more RNA species encompass the entire transcriptome of a cell or tissue sample of interest.

In step (b) of the methods of the present invention, the sample is subjected to a deamination reaction to obtain deaminated RNAs. Means and methods for the deamination of RNA are not particularly limited and are known in the art. Suitable deamination reactions include oxidative deamination reactions, and can be chemical or enzymatic deamination reactions.

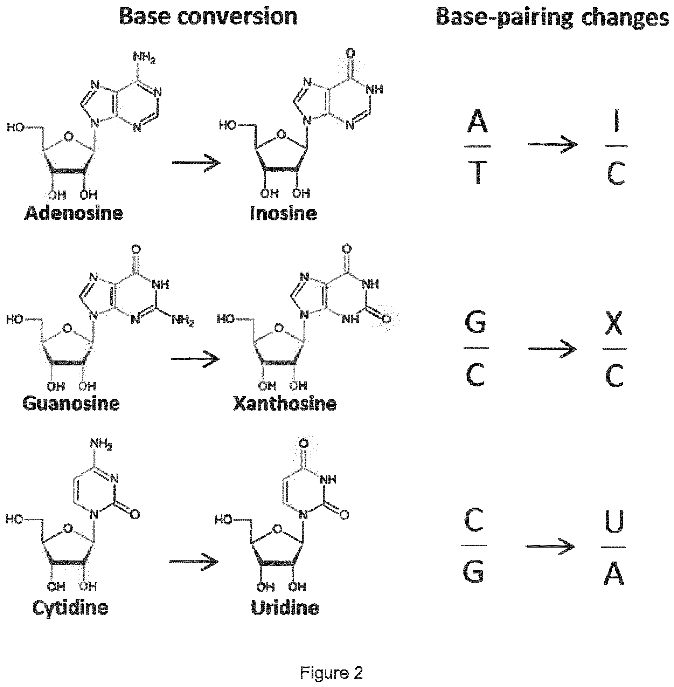

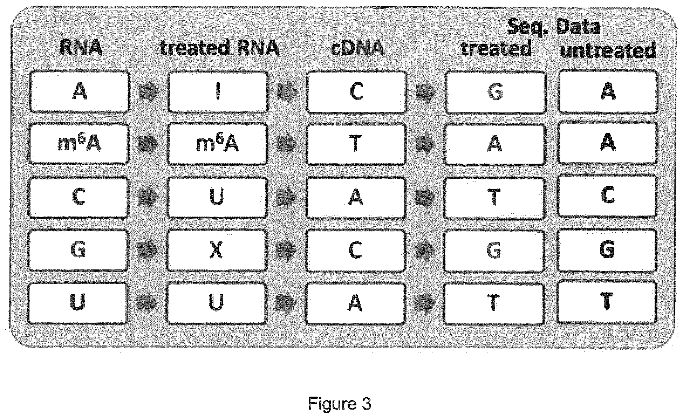

Deamination converts adenosine (A) to inosine (I), guanosine (G) to xanthosine (X), and cytidine (C) to uridine (U). However, uridine (U) is not converted by deamination, and, most importantly for the present invention, m6A and m4C, although converted by deamination to $N6$-methyl-$N6$-nitroso-adenosine and $N4$-methyl-$N4$-nitroso-cytidine, respectively, are still transcribed to 2'-deoxyribothymidine (dT) and 2'-deoxyriboguanosine (dG), respectively, in subsequent step (c) of the methods of the present invention (Figs. 2 and 3).

In step (c) of the methods of the present invention, the deaminated RNAs obtained in step (b) are subjected to reverse transcription using a reverse transcriptase (RT) to obtain respective cDNAs. Means for the reverse transcription of RNA into cDNA are not particularly limited and are known in the art. During reverse transcription, inosine (I), deriving from the deamination of an adenosine (A), is transcribed to 2'-deoxyribocytidine (dC), m6A, converted to N6-methyl-$N6$-nitroso-adenosine by the deamination, is transcribed to 2'-deoxyribothymidine (dT), m4C, converted to $N4$-methyl-$N4$-nitroso-cytidine by the deamination, is transcribed to 2'-deoxyriboguanosine (dG), uridine, either deriving from the deamination of a cytidine (C) or from an uridine (U) remaining unchanged by the deamination, is transcribed to 2'-deoxyriboadenosine (dA), and xanthosine (X), deriving from the deamination of a guanosine (G), is transcribed to a 2'-deoxyribocytidine (dC) (Fig. 3).

In specific embodiments, the generation of cDNAs from the deaminated RNAs obtained in step (b) of the methods of the present invention encompasses (i) the 5'-/3'-dephosphorylation of the deaminated RNAs, e.g. using a phosphatase, (ii) the 5'-phosphorylation of the dephosphorylated, deaminated RNAs, e.g. using a polynucleotide kinase, (iii) the purification of the phosphorylated RNAs, e.g. using commercially available RNA purification kits, (iv) the 3' ligation of adaptor oligonucleotides, e.g. adaptor oligonucleotides that facilitate subsequent sequencing steps, (v) the hybridization of suitable reverse transcription primers, (vi) the 5' ligation of adaptor oligonucleotides, e.g. adaptor oligonucleotides that facilitate subsequent sequencing steps, and (vii) the actual reverse transcription, e.g. using a reverse transcriptase derived from MMLV (Moloney Murine Leukemia Virus) reverse transcriptase and having reduced RNase H activity and increased thermostability. Respective methods are not particularly limited and are known in the art. Further, suitable reverse transcriptases are known in the art and include e.g. ProtoScript® II Reverse Transcriptase (New England BioLabs GmbH, Frankfurt am Main, Germany) and SuperScript IV (Thermo Fischer Scientific GmbH, Schwerte, Germany). Further, generation of cDNAs may encompass the amplification of obtained cDNAs by polymerase chain reaction (PCR), e.g. using one or more high fidelity DNA polymerase(s) derived from Taq (*Thermus aquaticus*) DNA polymerase. Suitable DNA polymerases or DNA polymerase mixtures are known in the art and include e.g. LongAmp® Taq DNA polymerase (New England BioLabs GmbH, Frankfurt am Main, Germany). Moreover, PCR products obtained in this step may be purified, e.g. using commercially available DNA purification kits.

In step (d) of the methods of the present invention, the cDNAs obtained in step (c) are sequenced. Means for sequencing DNA are not particularly limited and are known in the art. They include for example high throughput sequencing (Next-Generation-Sequencing) methods known in the art.

In specific embodiments, sequencing is done by a sequencing-by-synthesis method, e.g. a sequencing-by-synthesis method with bridge amplification, such as the Illumina dye sequencing method known in the art. In such embodiments, adaptors for 3' and 5' ligation, primers for reverse transcription, and primers for amplification of cDNAs are preferably suitably selected for the sequencing method used.

In other specific embodiments, sequencing is done by Nanopore sequencing as known in the art. In such embodiments, prior amplification of cDNAs is not necessary.

As shown in Figure 3, sequencing can yield, for each position, the following results. For an adenosine (A) in the RNA (deamination: I, reverse transcription: dC), sequencing data shows a 2'-deoxyriboguanosine (dG). For an m6A in the RNA (deamination: *N*6-methyl-*N*6-nitroso-adenosine, reverse transcription: dT), sequencing data shows a 2'-deoxyriboadenosine (dA). For a cytidine (C) in the RNA (deamination: U, reverse transcription: dA), sequencing data shows a 2'-deoxyribothymidine (dT). For an m4C in the RNA (deamination: *N*4-methyl-*N*4-nitroso-cytidine, reverse transcription: dG), sequencing data shows a 2'-deoxyribocytidine (dC). For a guanosine (G) in the RNA (deamination: X, reverse transcription: dC), sequencing data shows a 2'-deoxyriboguanosine (dG). For an uridine (U) in the RNA (deamination: U, reverse transcription: dA), sequencing data shows a 2'-deoxyribothymidine (dT).

Accordingly, an adenosine (A) in the sequencing data can only be the result of an m6A having been present in this position in the RNA. Likewise, a cytidine (C) in the sequencing data can only be the result of an m4C having been present in this position in the RNA. Thus, any dA or dC in the sequencing data indicates the presence of the respective m6A or m4C in that position. Therefore, in step (e) of the methods of the present invention, the presence of m6A at a given position in the RNA is determined, in case the sequencing data show a 2'-deoxyriboadenosine (dA) in the corresponding position, or the presence of m4C at a given position in the RNA is determined, in case the sequencing data show a 2'-deoxyribocytidine (dC) in the corresponding position.

In a first embodiment, the deamination reaction in step (b) of the methods of the present invention is performed in a manner to result in completely deaminated RNAs (complete deamination). Means for completely deaminating RNAs are not particularly limited and are known in the art. They include for example the incubation of RNA in the presence of about 1 M NaOAc/HOAc (pH 3.5) and about 1 M $NaNO_2$ for about 30 minutes at about 70°C.

In a specific embodiment, the complete deamination reaction is performed

    (i) at a pH of 2.5 to 4.5, and
    (ii) at a temperature of 60°C to 80°C, and
    (iii) for a duration of 15 to 45 minutes.

In this context, it should be noted that the sequencing step (d) of the methods of the present invention usually provides a number of test sequences for a given RNA or stretch of RNA. This is due to the presence of usually more than one copy of a given RNA in the sample.

Further, it should be noted that for a given RNA species having a position wherein an adenosine is an m6A, this position may not be methylated in all RNA molecules, but only in a certain portion of said RNA molecules, the remaining portion having an A in this position. Thus, for a given RNA species having in a first position an A that is never methylated, and having in a second position an A that is methylated in a certain portion of RNA molecules, the test

4

sequences show in the first position a G in said position due to complete deamination of said position. In the above second position, a portion of test sequences may show an A due to the presence of m$^6$A in said position, whereas another portion of test sequences may show a G in said position due to the fact that said position had not been methylated. Thus, any A in a test sequence indicates the presence of m$^6$A in said position in the RNA, at least in a portion of RNA molecules, wherein the proportion of A in the test sequences, as opposed to G in said position, directly correlates with the proportion of RNA molecules in which said position had been methylated. The above analogously applies to cytidines and m$^4$C.

However, complete deamination can negatively impact the methods of the present invention in terms of the loss of RNA template, difficult cDNA generation and/or difficult sequencing, due to the relatively harsh conditions during complete deamination. Therefore, in a second embodiment, the deamination reaction in step (b) of the methods of the present invention is performed in a manner to result in only partially deaminated RNAs (partial deamination). In this context, the term "partial deamination" as used herein refers to deamination of 5 to 80%, preferably 20 to 60%, more preferably 30 to 50%, more preferably 35 to 45%, and most preferably about 40% of the amine-containing nucleobases. By way of example, the term "partial deamination" may refer to the deamination of e.g. 80% of the total number of adenosines (A), cytidines (C), and guanosines (G) in a given RNA. Means for the partial deamination of RNA are not particularly limited and are known in the art. They include for example the incubation of RNA in the presence of about 1 M NaOAc/HOAc (pH 4.0) and about 1 M NaNO$_2$ for about 20 minutes at about 50°C.

In a specific embodiment, the partial deamination reaction is performed

(i) at a pH of 3 to 5, and
(ii) at a temperature of 40°C to 60°C, and
(iii) for a duration of 1 to 30 minutes.

In case the methods of the present invention use partial deamination in step (b), several adaptations of said methods are necessary, since in this case, a 2'-deoxyriboadenosine (dA) or 2'-deoxyribocytidine (dC) in the sequencing data no longer clearly indicates the presence of m$^6$A and m$^4$C, respectively, in the corresponding position in the RNA, but may be the result of the fact that an adenosine (A) or cytidine (C) in this given position in the RNA might not have been deaminated.

Accordingly, in respective embodiments of the methods of the present invention, the deamination reaction is performed in a manner to result in partially deaminated RNAs, and
step (e) comprises the steps

(e1) aligning the sequences determined in step (d) (test sequences) with respective reference sequences,
(e2) determining for each A in a reference sequence the number of test sequences showing a dA in the respective position (n(A)) and the number of test sequences showing a dG in the respective position (n(G)), and/or determining for each C in a reference sequence the number of test sequences showing a dC in the respective position (n(C)) and the number of test sequences showing a dT in the respective position (n(T)),
(e3) determining for each of the respective positions an A-to-G-conversion ratio and/or a C-to-T-conversion ratio, and
(e4) determining

(i) the presence of m$^6$A at a given position in the RNA, in case the respective A-to-G-conversion ratio is significantly lower compared to the A-to-G-conversion ratios in other positions of the test sequence, or
(ii) the presence of m$^4$C at a given position in the RNA, in case the C-toT-conversion ratio is significantly lower compared to the C-to-T-conversion ratios in other positions of the test sequence.

In specific embodiments, the reference sequences used in step (e1) of the methods of the present invention can be derived from a database, or any other source of nucleotide sequence data. Respective reference sequences can be found in a database or other source of nucleotide sequence data by way of alignment of the test sequences, e.g. alignment as defined hereinafter. Alternatively, the reference sequences used in step (e1) of the methods of the present invention are generated in the methods themselves, wherein
in step (b), a first aliquot of said sample is subjected to said deamination reaction, whereas a second aliquot of said sample is left untreated as a reference,
in step (c), said deaminated RNAs and the untreated reference RNAs are subjected to reverse transcription using a reverse transcriptase to obtain respective cDNAs and reference cDNAs, and
in step (d), sequencing of said cDNAs and reference cDNAs yields sequencing data for the cDNAs (test sequences) and reference cDNAs (reference sequences). In specific embodiments, these reference cDNAs are used as inputs for *de novo* sequencing tools. These latter reassemble the reference RNA sequence, which is used for the alignment of the test sequences.

In this context, it should again be noted that the sequencing step (d) of the methods of the present invention usually provides a number of test sequences for a given RNA or stretch of RNA. This is due to the presence of usually more than one copy of a given RNA in the sample.

Further, it should again be noted that for a given RNA species having a position wherein an adenosine is an m6A, this position may not be methylated in all RNA molecules, but only in a certain portion of said RNA molecules, the remaining portion having an A in this position. Thus, for a given RNA species having in a first position an A that is never methylated, and having in a second position an A that is methylated in a certain portion of RNA molecules, a portion of test sequences may show in the first position a dA due to partial deamination, leaving said A aminated, and another portion of test sequences may show a dG in said position due to deamination of said position. In the above second position, a portion of test sequences may show a dA due to the presence of m6A in said position, whereas another portion of test sequences may show a dG in said position due to the fact that said position had not been methylated. In addition, in the above second position, in those RNA molecules that had not been methylated in said position, some test sequences may show dA due to partial deamination. However, RNA species having, at least to some extent, m6A in a certain position, can still be identified using the methods of the present invention and the embodiments detailed hereinafter. The above analogously applies to cytidines and m4C.

In specific embodiments, in the above step (e1) of the methods of the present invention, the alignment of the test sequences with the reference sequences uses the following matching rules:

(i) for 2'-deoxyribothymidine (dT) sites in the reference sequence, the matching of dT in the test sequence is allowed,
(ii) for 2'-deoxyriboguanosine (dG) sites in the reference sequence, the matching of dG in the test sequence is allowed,
(iii) for 2'-deoxyriboadenosine (dA) sites in the reference sequence, the matching of dA and dG in the test sequence is allowed, and
(iv) for 2'-deoxyribocytidine (dC) sites in the reference sequence, the matching of dC and dT in the test sequence is allowed.

Further, alignment may be based on the Smith-Waterman- and/or the Needleman-Wunsch algorithm known in the art.

Further, the A-to-G-conversion ratio and/or the C-to-T-conversion ratio for each position can be determined in step (e3) of the methods of the present invention as

$$R_{A>G} = \frac{n(G)}{n(A) + n(G)}$$

and

$$R_{C>T} = \frac{n(T)}{n(C) + n(T)}$$

wherein

$R_{A>G}$ is the A-to-G-conversion ratio,
$R_{C>T}$ is the C-to-T-conversion ratio,
$n(G)$ is the number of test sequences showing a dG in said position,
$n(A)$ is the number of test sequences showing an dA in said position,
$n(T)$ is the number of test sequences showing a dT in said position, and
$n(C)$ is the number of test sequences showing a dC in said position.

In specific embodiments, the A-to-G-conversion ratio or the C-to-T-conversion ratio in a given position is considered to be significantly lower compared to the A-to-G-conversion ratios or the C-to-T-conversion ratios in other positions of the test sequence (step (e4)), in case

(i) the signal-to-noise ratio (S/N-ratio) is above a default value, wherein

$$S/N\text{-}ratio_i = \frac{average(R_{A>G})}{R_{A>G}(i)}$$

or

$$S/N\text{-}ratio_i = \frac{average(R_{C>T})}{R_{C>T}(i)}$$

wherein

$S/N\text{-}ratio_i$ is the signal-to-noise ratio in a given position i,
$average(R_{A>G})$ is the average A-to-G-conversion ratio in the other positions of the test sequence,
$average(R_{C>T})$ is the average C-to-T-conversion ratio in the other positions of the test sequence,
$R_{A>G}(i)$ is the A-to-G-conversion ratio in position i, and
$R_{C>T}(i)$ is the C-to-T-conversion ratio in position i; and/or

(ii) the p-value as determined in a Student's t-test comparing the A-to-G-conversion ratio or the C-to-T-conversion ratio in a given position to the A-to-G-conversion ratios or the C-to-T-conversion ratios in other positions of the test sequence is below a default value.

In these embodiments, the default value for the S/N-ratio is preferably about 1.5 to about 3, more preferably about 2 to about 3, more preferably about 2.5 to about 3, more preferably about 3, and the default p-value in the Student's t-test is preferably about 0.001 (0.1%), about 0.01 (1%) or about 0.05 (5%). Further, the term "other positions of the test sequence" as used herein preferably relates to 2, 3, 4, 5, 6, 7, 8, 9, 10, or more, or all other positions of the test sequence.
According to some embodiments, the methods of the present invention further comprise

(i) a step of enriching m6A- and/or m4C-containing RNAs by immunoprecipitation in the sample provided in step (a) prior to step (b), and/or
(ii) a step of amplifying the cDNAs obtained in step (c) prior to sequencing in step (d), e.g. as described above.

Methods for enriching m6A- and/or m4C-containing RNAs by immunoprecipitation, using antibodies, antibody fragments or antibody mimetics specifically binding to m6A or m4C, are not particularly limited and are known in the art. They include for example methyl-RNA immunoprecipitation protocol (Me-RIP) methods known in the art.
Further, methods for the amplification of cDNAs are not particularly limited and are known in the art. They include any suitable polymerase chain reaction (PCR)-based techniques known in the art, e.g. as described above.
In preferred embodiments of the methods of the present invention, the nucleotide modification to be detected is m6A.
In a related aspect, the present invention relates to an analogous method for detecting the position of a nucleotide modification, selected from the group consisting of $N^6$-methyl-2'-deoxyriboadenosine (m6dA) and $N^4$-methyl-2'-deoxyribocytidine (m4dC) in one or more DNA species, comprising the steps:

(a) providing a sample comprising said one or more DNA species,
(b) subjecting said sample to a deamination reaction to obtain deaminated DNAs,
(c) sequencing said DNAs,
(d) rewriting the sequencing data as their reverse complement, and
(e) determining the presence of m6dA at a given position in the DNA, in case the rewritten sequencing data show a 2'-deoxyriboadenosine (dA) in the corresponding position, or the presence of m4dC at a given position in the DNA, in case the rewritten sequencing data show a 2'-deoxyribocytidine (dC) in the corresponding position.

As used herein, the term "detecting the position of a nucleotide modification, selected from the group consisting of m6dA and m4dC in one or more DNA species" relates to the determination of the presence or absence and the location of one of said modifications for every nucleoside in a DNA sequence. In particular, the methods of the present invention do not require any predetermination of a specific DNA sequence or a specific location in a given sequence. Instead, said methods allow the analysis of any given DNA-containing sample. In case any DNA sequence in said sample contains an m6dA or m4dC, the presence and exact location in of said m6dA or m4dC in said sequence can be determined.
As used herein, the term "sample containing one or more DNA species" relates to any sample containing, or being suspected of containing, any one or more DNAs. In specific embodiments, said one or more DNA species encompass the entire genome of a cell or tissue sample of interest.
In step (b) of the methods of the present invention, the sample is subjected to a deamination reaction to obtain deaminated DNAs. Means and methods for the deamination of DNA are not particularly limited and are known in the art. Suitable deamination reactions include oxidative deamination reactions, and can be chemical or enzymatic deamination reactions.

Deamination converts 2'-deoxyriboadenosine (dA) to 2'-deoxyriboinosine (dI), 2'-deoxyriboguanosine (dG) to 2'-deoxyriboxanthosine (dX), and 2'-deoxyribocytidine (dC) to 2'-deoxyribouridine (dU). However, 2'-deoxyribothymidine (dT) is not converted by deamination. Most importantly for the present invention, m6dA and m4dC, although converted by deamination to N6-methyl-N6-nitroso-deoxyriboadenosine and N4-methyl-N4-nitroso-deoxyribocytidine, respectively, are still replicated by a DNA polymerase into 2'-deoxyribothymidine (dT) and 2'-deoxyriboguanosine (dG), respectively, in the subsequent step (c) of the methods of the present invention.

In steps (c) and (d) of the methods of the present invention, the deaminated DNAs obtained in step (b) are first sequenced and then the sequencing data are rewritten as their reverse complement. Means for rewriting sequencing data as their reverse complement are not particularly limited and are known in the art. During the above steps (c) and (d),

> (i) 2'-deoxyriboinosine (dI), deriving from the deamination of a 2'-deoxyriboadenosine (dA), is sequenced as 2'-deoxyribocytidine (dC), and then rewritten as a 2'-deoxyriboguanosine (dG);
> (ii) m6dA, converted to N6-methyl-N6-nitroso-deoxyriboadenosine by the deamination, is sequenced as 2'-deoxyribothymidine (dT), and then rewritten as a 2'-deoxyriboadenosine (dA);
> (iii) m4dC, converted to N4-methyl-N4-nitroso-deoxyribocytidine by the deamination, is sequenced as 2'-deoxyriboguanosine (dG), and then rewritten as a 2'-deoxyribocytidine (dC);
> (iv) 2'-deoxyribouridine (dU), deriving from the deamination of a 2'-deoxyribocytidine (dC) is sequenced as 2'-deoxyriboadenosine (dA), and then rewritten as a 2'-deoxyribothymidine (dT);
> (v) 2'-deoxyriboxanthosine (dX), deriving from the deamination of a 2'-deoxyriboguanosine (dG), is sequenced as 2'-deoxyribocytidine (dC), and then rewritten as a 2'-deoxyriboguanosine (dG); and
> (vi) 2'-deoxyribothymidine, remaining unchanged by the deamination, is sequenced as 2'-deoxyriboadenosine, and then rewritten as 2'-deoxyribothymidine.

Means for sequencing DNA are not particularly limited and are known in the art. They include for example high throughput sequencing (Next-Generation-Sequencing) methods known in the art.

In specific embodiments, sequencing is done by a sequencing-by-synthesis method, e.g. a sequencing-by-synthesis method with bridge amplification, such as the Illumina dye sequencing method known in the art. In such embodiments, adaptors for 3' and 5' ligation, and primers for amplification of DNAs are preferably suitably selected for the sequencing method used.

In other specific embodiments, sequencing is done by Nanopore sequencing as known in the art.

According to this aspect of the present invention, a 2'-deoxyriboadenosine (dA) in the rewritten sequencing data can only be the result of an m6dA having been present in this position in the DNA. Further, a 2'-deoxyribocytidine (dC) in the rewritten sequencing data can only be the result of an m4dC having been present in this position in the DNA. Thus, any dA in the rewritten sequencing data indicates the presence of m6dA in that position, and any dC in the rewritten sequencing data indicates the presence of m4dC in that position. Therefore, in step (e) of the methods of the present invention, the presence of m6dA at a given position in the DNA is determined, in case the rewritten sequencing data show a 2'-deoxyriboadenosine (dA) in the corresponding position. Further, the presence of m4dC at a given position in the DNA is determined, in case the rewritten sequencing data show a 2'-deoxyribocytidine (dC) in the corresponding position.

In a first embodiment, the deamination reaction in step (b) of the methods of the present invention is performed in a manner to result in completely deaminated DNAs (complete deamination). Means for completely deaminating DNAs are not particularly limited and are known in the art. They include for example the incubation of DNA in the presence of about 1 M NaOAc/HOAc (pH 4.0) and about 1 M $NaNO_2$ for about 60 minutes at about 70°C.

In a specific embodiment, the partial deamination reaction is performed

> (i) at a pH of 3 to 5, and
> (ii) at a temperature of 60°C to 80°C, and
> (iii) for a duration of 45 to 75 minutes.

However, complete deamination can negatively impact the methods of the present invention in terms of the loss of DNA template and/or difficult sequencing, due to the relatively harsh conditions during complete deamination. Therefore, in a second embodiment, the deamination reaction in step (b) of the methods of the present invention is performed in a manner to result in only partially deaminated DNAs (partial deamination). In this context, the term "partial deamination" as used herein refers to deamination of 5 to 80%, preferably 20 to 60%, more preferably 30 to 50%, more preferably 35 to 45%, and most preferably about 40% of the amine-containing nucleobases. Means for the partial deamination of DNA are not particularly limited and are known in the art. They include for example the incubation of DNA in the presence of about 1 M NaOAc/HOAc (pH 4.5) and about 1 M $NaNO_2$ for about 30 minutes at about 60°C.

In a specific embodiment, the partial deamination reaction is performed

(i) at a pH of 3.5 to 5.5, and
(ii) at a temperature of 50°C to 70°C, and
(iii) for a duration of 10 to 40 minutes.

In case the methods of the present invention use partial deamination in step (b), several adaptations of said methods are necessary, since in this case, a 2'-deoxyriboadenosine (dA) or 2'-deoxyribocytidine (dC) in the rewritten sequencing data no longer clearly indicates the presence of $m^6dA$ and $m^4dC$, respectively, in the corresponding position in the DNA, but may be the result of the fact that an 2'-deoxyriboadenosine (dA) or 2'-deoxyribocytidine (dC) in this given position in the DNA might not have been deaminated.

Accordingly, in respective embodiments of the methods of the present invention, the deamination reaction is performed in a manner to result in partially deaminated DNAs, and

step (e) comprises the steps

(e1) aligning the rewritten sequences determined in step (d) (test sequences) with respective reference sequences,
(e2) determining for each dA in a reference sequence the number of test sequences showing a dA in the respective position (n(A)) and the number of test sequences showing a dG in the respective position (n(G)), and/or determining for each dC in a reference sequence the number of test sequences showing a dC in the respective position (n(C)) and the number of test sequences showing an dT in the respective position (n(T)),
(e4) determining for each of the respective positions a A-to-G-conversion ratio and/or a C-to-T-conversion ratio, and
(e5) determining

(i) the presence of $m^6dA$ at a given position in the DNA, in case the respective A-to-G-conversion ratio is significantly lower compared to the A-to-G-conversion ratios in other positions of the test sequence, or
(ii) the presence of $m^4dC$ at a given position in the DNA, in case the C-toT-conversion ratio is significantly lower compared to the C-to-T-conversion ratios in other positions of the test sequence.

In specific embodiments, the reference sequences used in step (e1) of the methods of the present invention can be derived from a database, or any other source of nucleotide sequence data. Respective reference sequences can be found in a database or other source of nucleotide sequence data by way of alignment of the test sequences, e.g. alignment as defined hereinafter. Alternatively, the reference sequences used in step (e1) of the methods of the present invention are generated in the methods themselves, wherein

in step (b), a first aliquot of said sample is subjected to said deamination reaction, whereas a second aliquot of said sample is left untreated as a reference,

in step (c), sequencing of said DNAs and reference DNAs yields sequencing data for the DNAs (test sequences) and reference DNAs (reference sequences). In specific embodiments, these reference DNAs are used as inputs for *de novo* sequencing tools. These latter reassemble the reference DNA sequence, which is used for the alignment of the test sequences.

In this context, it should again be noted that for a given DNA species having a position wherein an 2'-deoxyriboadenosine is an $m^6dA$, this position may not be methylated in all DNA molecules, but only in a certain portion of said DNA molecules, the remaining portion having a dA in this position. Thus, for a given DNA species having in a first position a dA that is never methylated, and having in a second position a dA that is methylated in a certain portion of DNA molecules, a portion of test sequences may show in the first position a dA due to partial deamination, leaving said dA aminated, and another portion of test sequences may show a dG in said position due to deamination of said position. In the above second position, a portion of test sequences may show a dA due to the presence of $m^6dA$ in said position, whereas another portion of test sequences may show a dG in said position due to the fact that said position had not been methylated. In addition, in the above second position, in those DNA molecules that had not been methylated in said position, some test sequences may show dA due to partial deamination. However, DNA species having, at least to some extent, $m^6dA$ in a certain position, can still be identified using the methods of the present invention and the embodiments detailed hereinafter. The above analogously applies to 2'-deoxyribocytidines and $m^4dC$.

In specific embodiments, in the above step (e1) of the methods of the present invention, the alignment of the test sequences with the reference sequences uses the following matching rules:

(i) for 2'-deoxyribothymidine (dT) sites in the reference sequence, the matching of dT in the test sequence is allowed,
(ii) for 2'-deoxyriboguanosine (dG) sites in the reference sequence, the matching of dG in the test sequence is allowed,
(iii) for 2'-deoxyriboadenosine (dA) sites in the reference sequence, the matching of dA and dG in the test sequence is allowed, and
(iv) for 2'-deoxyribocytidine (dC) sites in the reference sequence, the matching of dC and dT in the test sequence is allowed.

Further, alignment may be based on the Smith-Waterman- and/or the Needleman-Wunsch algorithm known in the art.

Further, the A-to-G-conversion ratio and/or the C-to-T-conversion ratio for each position can be determined in step (e4) of the methods of the present invention as

$$R_{A>G} = \frac{n(G)}{n(A) + n(G)}$$

and

$$R_{C>T} = \frac{n(T)}{n(C) + n(T)}$$

wherein

$R_{A>G}$ is the A-to-G-conversion ratio,
$R_{C>T}$ is the C-to-T-conversion ratio,
$n(G)$ is the number of test sequences showing a dG in said position,
$n(A)$ is the number of test sequences showing an dA in said position,
$n(T)$ is the number of test sequences showing a dT in said position, and
$n(C)$ is the number of test sequences showing a dC in said position.

In specific embodiments, the A-to-G-conversion ratio or the C-to-T-conversion ratio in a given position is considered to be significantly lower compared to the A-to-G-conversion ratios or the C-to-T-conversion ratios in other positions of the test sequence (step (d5)), in case

(i) the signal-to-noise ratio (S/N-ratio) is above a default value, wherein

$$S/N\text{-}ratio_i = \frac{average(R_{A>G})}{R_{A>G}(i)}$$

or

$$S/N\text{-}ratio_i = \frac{average(R_{C>T})}{R_{C>T}(i)}$$

wherein

$S/N\text{-}ratio_i$ is the signal-to-noise ratio in a given position i,
$average(R_{A>G})$ is the average A-to-G-conversion ratio in the other positions of the test sequence,
$average(R_{C>T})$ is the average C-to-T-conversion ratio in the other positions of the test sequence,
$R_{A>G}(i)$ is the A-to-G-conversion ratio in position i, and
$R_{C>T}(i)$ is the C-to-T-conversion ratio in position i; and/or

(ii) the p-value as determined in a Student's t-test comparing the A-to-G-conversion ratio or the C-to-T-conversion ratio in a given position to the A-to-G-conversion ratios or the C-to-T-conversion ratios in other positions of the test sequence is below a default value.

In these embodiments, the default value for the S/N-ratio is preferably about 1.5 to about 3, more preferably about 2 to about 3, more preferably about 2.5 to about 3, more preferably about 3, and the default p-value in the Student's t-test is preferably about 0.001 (0.1%), about 0.01 (1%) or about 0.05 (5%). Further, the term "other positions of the test sequence" as used herein preferably relates to 2, 3, 4, 5, 6, 7, 8, 9, 10, or more, or all other positions of the test sequence.

According to some embodiments, the methods of the present invention further comprise

(i) a step of enriching $m^6dA$- and/or $m^4dC$-containing DNAs by immunoprecipitation in the sample provided in step (a) prior to step (b), and/or
(ii) a step of amplifying the DNAs prior to sequencing in step (c).

Methods for enriching $m^6dA$- and/or $m^4dC$-containing DNAs by immunoprecipitation, using antibodies, antibody fragments or antibody mimetics specifically binding to $m^6dA$ or $m^4dC$, are not particularly limited and are known in the art.

Further, methods for the amplification of DNAs are not particularly limited and are known in the art. They include any suitable polymerase chain reaction (PCR)-based techniques known in the art, e.g. as described above.

In preferred embodiments of the methods of the present invention, the nucleotide modification to be detected is $m^6dA$.

Otherwise, the methods according to this aspect of the present invention are largely analogous to the above methods using RNA, wherein the step of reverse transcription of RNA into cDNA is, however, lacking. Thus, definitions and embodiments described above for the methods of the present invention using RNA, where applicable, apply in an analogous manner.

In specific embodiments, the methods of the present invention can not only be used for detecting the position of $m^6A/m^6dA$ or $m^4C/m^4dC$ modification in one or more RNA or DNA species, but also for the quantification of said modifications, i.e., for determining the relative amount of copies of a specific sequence in a sample in which a particular (2'-deoxyribo)adenosine (A/dA) or (2'-deoxyribo)cytidine (C/dC) is methylated.

In these embodiments, the methods of the present invention (i) do not comprise a step of enriching $m^6A/m^6dA$- and/or $m^4C/m^4dC$-containing RNAs or DNAs by immunoprecipitation in the sample provided in step (a), prior to step (b), (ii) use a deamination reaction in step (b) that is performed in a manner to result in partially deaminated RNAs or DNAs, and (iii) use Student's t-test for determining whether the A-to-G-conversion ratio and/or the C-to-T-conversion ratio in a given position is significantly lower compared to the A-to-G-conversion ratios and/or the C-to-T-conversion ratios in other positions of the test sequence. Further, in these embodiments the methods of the present invention comprise a step of correlating the p-value score in a given position to the p-value score in said position obtained for samples containing known amounts of methylated RNA or DNA in unmethylated RNA or DNA.

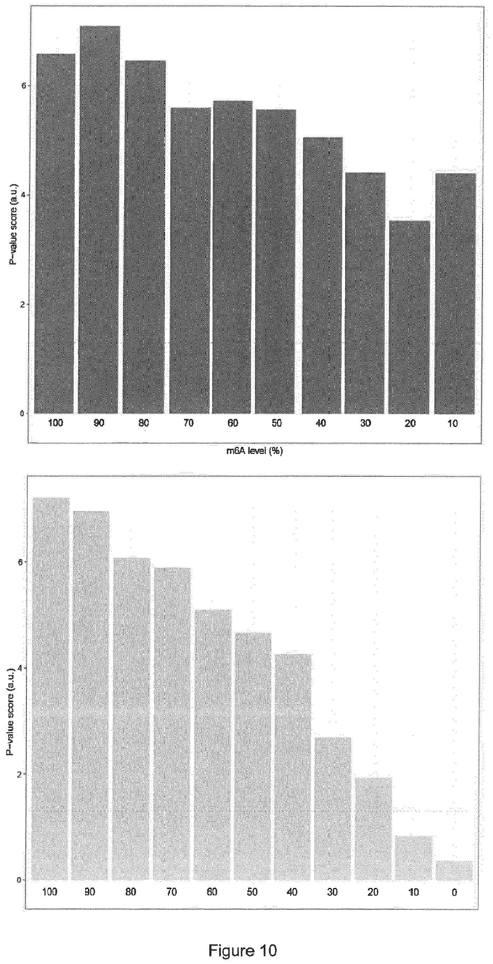

In particular, referring to the lower panel of Figure 10, a linear correlation of the p-value score with the relative amount of methylated RNA or DNA can be observed. Thus, for any given sequence of interest, a calibration curve can be established using samples having known relative amounts of methylated RNA or DNA. A test sample of interest can then be analyzed and the relative amount of methylation determined by correlating the p-value score of said sample to this calibration curve. Moreover, from the A-to-G-conversion graphs or C-to-T-conversion graphs, $m^6A/m^6dA$ or $m^4C/m^4dC$ position results in a drop. The height of this drop is directly correlated to the $m^6A/m^6dA$ or $m^4C/m^4dC$ levels and can be quantified. If the drop is quantified, then the $m^6A/m^6dA$ or $m^4C/m^4dC$ proportion in the samples can be quantified.

The term "about" as used herein is intended to by a modifier of the specified value of $\pm 10\%$, preferably $\pm 8\%$, $\pm 6\%$, $\pm 5\%$, $\pm 4\%$, $\pm 3\%$, $\pm 2\%$, $\pm 1.5\%$, $\pm 1\%$, or $\pm 0.5\%$. By way of example, the term "about 1 M $NaNO_2$" can refer to a concentration range of 0.9 M $NaNO_2$ to 1.1 $NaNO_2$.

The present invention provides methods for the detection of the presence and position of $m^6A$ and $m^4C$ nucleotide modifications in complex RNA samples.

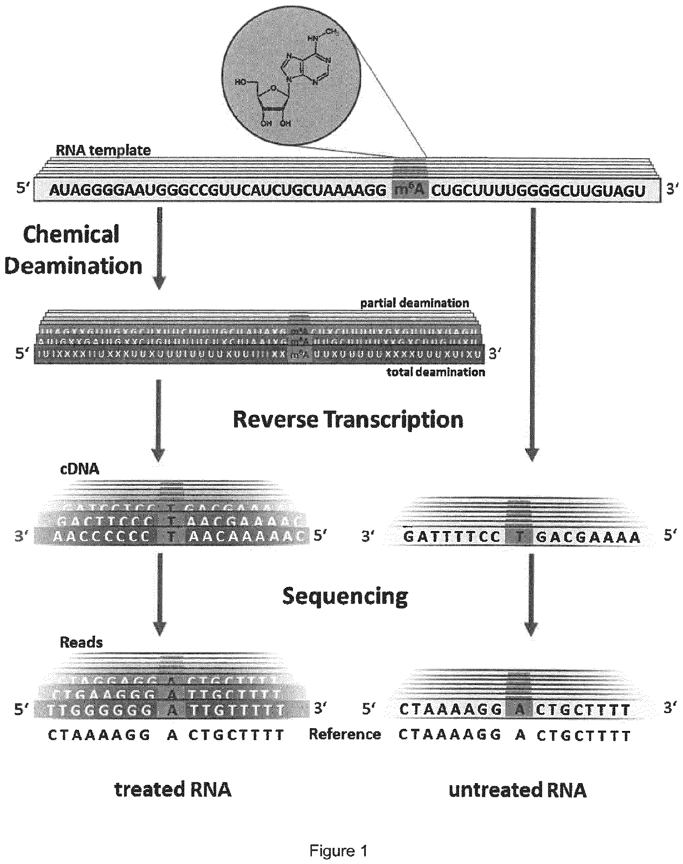

$m^6A$ is a RNA modification that cannot be distinguished from unmethylated adenosines in common RNA sequencing. Since the additional methylation has no influence on the reverse transcription, $m^6A$ is read by a reverse transcriptase like an unmethylated adenosine and reverse transcribed into thymidine in cDNA, resulting in loss of information. After subsequent amplification by PCR, the thymidines are detected in sequencing and presented as adenosines in sequencing data. $m^6A$ and adenosines differ only in the secondary and primary amine respectively. On this basis, a way had to be found to carry out a selective and specific pretreatment of the RNA to further ensure distinctness in sequencing data. The present invention provides a sophisticated combination of chemical deamination, adapted library preparation, strongly customized deep sequencing protocols and advanced bioinformatics to overcome these limitations and accurately predict e.g. $m^6A$ positions in RNA templates (Fig. 1).

Herein presented is a detection method for RNA modifications such as $m^6A$, based on an essential combination of a precisely harmonized chemical deamination pretreatment of the RNA followed by an adapted and well-selected library preparation protocol, subsequent Illumina sequencing and an advanced bioinformatic analysis. In order to distinguish $m^6A$ from unmethylated adenosines in sequencing data and to achieve an accurate determination of the $m^6A$ position in RNA templates, a base conversion has to be performed. This conversion is achieved in the method of the present invention by chemical deamination. The reaction requires special conditions and an innovative bioinformatic data processing, which leads to the successful detection of $m^6A$ positions using self-developed algorithms. First studies on DNA templates suggest that m6dA detection in DNA is also a successful option and offers the potential to extend the analytical spectrum of this method.

In the rapidly developing field of modification analytics, where the main focus in the future will be on the investigation of the biological significance of modifications with respect to biological processes and their relation to diseases, the detailed mapping of modified sites in nucleic acids will be a crucial factor. This concerns not only academic research but also numerous young biotechnology companies that have specialized on developing small molecule inhibitors of RNA-modifying enzymes or optimized protein-coding mRNA with targeted *in vivo* delivery to individualize cancer immunotherapy, for prophylactic vaccines and protein replacement therapies. The RNA modification $m^6A$ in particular is currently attracting great attention, as the previously known $m^6A$ regulators, the writer, reader and eraser proteins, appear to play a decisive role in various disease processes, in particular in several types of cancer. The clinical significance of these findings lies in the fact that they can be used for prognosis, diagnosis and in some cases also for therapeutic purposes. Further, according to the strongly increasing amount of recent publications, it can be assumed that interest and developments in this field of research will gain even more momentum in the coming years. Therefore, the RNA community awaits validation methods that can be easily and cost-effectively established and applied in any laboratory worldwide. The $m^6A$ detection method of the present invention shows the potential to fulfill these requirements and expectations.

The figures show:

Figure 1:
Extended principle of chemical deamination treatment showing the enabled distinctiveness of unmethylated adenosines and $m^6A$. On the left side the pretreatment and its effects on the reverse transcription and sequencing of the $m^6A$-bearing RNA template is shown. Total or partial deamination induce base-conversion at A, C and G sites to I, U and X and thereby subsequently lead to changes in the base-pairing. This allows changes to be detected in the reads of the sequencing data set in comparison to the reference. The occurring dG content at unmethylated adenosine sites can be used to differentiate $m^6A$. In comparison, on the right side the reverse transcription and sequencing of the untreated RNA template is shown. A differentiation between A and $m^6A$ is not possible in the sequencing data, both are occurring as dA in the reads.

Figure 2:
Base conversion through chemical deamination and its effects on base-pairing. Adenosine (A), Guanosine (G) and Cytidine (C) are converted to Inosine (I), Xanthosine (X) and Uridine (U). Uridine itself remains unchanged.

Figure 3:
Base conversion and its effects on the different nucleotides during treatment, library preparation and sequencing.

Figure 4:
(I) Typical HPLC chromatogram for treated RNA with certain conditions, (II) Bar chart showing the nucleoside ratio [%] for deamination after 1, 3, 5, 10, 20 and 30 min treatment with certain conditions.

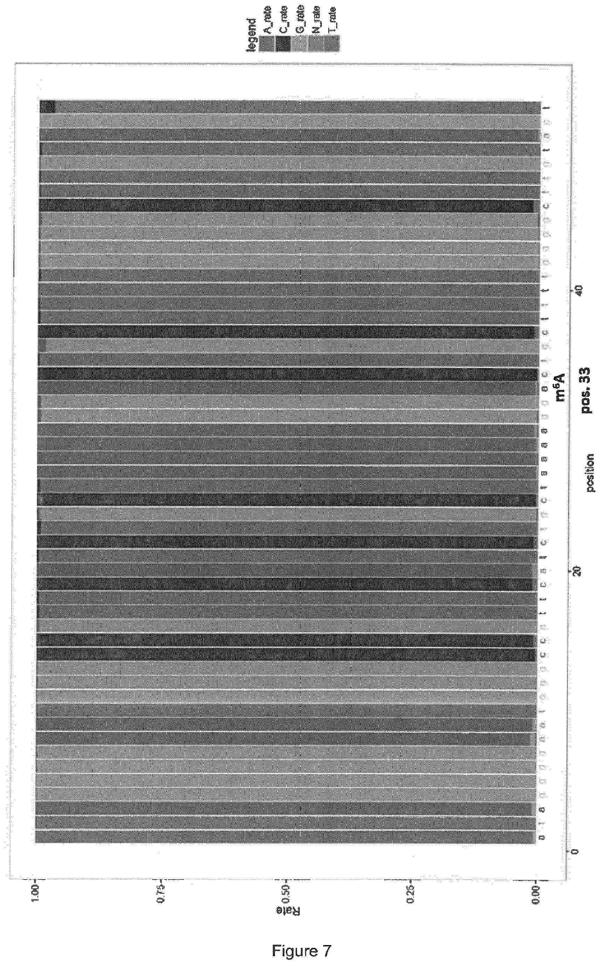

Figure 5:
Histogram showing the base composition (amount for each nucleotide) for each position of the deaminated RNA oligo template at certain conditions (for comparison with untreated RNA oligo template (reference) see Fig. 7). The increasing guanosine (orange) content at adenosine sites is visible, in contrast to the stable adenosine (green) content at the $m^6A$ site.

Figure 6:
Histogram showing the A-to-G-conversion for adenosine sites of the deaminated RNA oligo template at certain conditions. The increasing guanosine content at unmethylated adenosine sites in contrast to the $m^6A$ site is visible.

Figure 7:
Histogram showing the base composition (amount for each nucleotide) for each position of the untreated RNA oligo template (reference). There is no discrimination between A and $m^6A$ sites possible.

Figure 8:
Exemplary alignment matrix. The first line boxed in red correspond to the reference sequence (from a nucleotide database), the other lines to the test sequences (cDNAs).

Figure 9:

S/N-ratios at position 33 of samples containing defined mixtures of the $m^6A$-containing RNA according to SEQ ID NO: 1 and the respective non-methylated RNA according to SEQ ID NO: 2, with (upper panel) and without (lower panel) immunoprecipitation.

Figure 10:
Student's t-test p-value scores at position 33 of samples containing defined mixtures of the $m^6A$-containing RNA according to SEQ ID NO: 1 and the respective non-methylated RNA according to SEQ ID NO: 2, with (upper panel) and without (lower panel) immunoprecipitation.

The present application discloses the following sequences:

SEQ ID NO: 1
$m^6A$-containing RNA oligonucleotide
AUAGGGGAAUGGGCCGUUCAUCUGCUAAAAGG($m^6A$)CUGCUUUUGGGGCUUGUAGU

SEQ ID NO: 2
non-methylated RNA oligonucleotide corresponding to SEQ ID NO: 1
AUAGGGGAAUGGGCCGUUCAUCUGCUAAAAGG**A**CUGCUUUUGGGGCUUGUAGU

The present invention will be further illustrated by the following example without being limited thereto.

**Examples**

Material and methods:

*Deamination protocol*

Materials:

- 3 M $CH_3COONa$ (Merck, 137046) / $CH_3COOH$ (Sigma Aldrich, 49199) buffer pH 3.5/4.0/4.5/5.0
- 5 M $NaNO_2$ (Merck, 106544)
- 0.5/5 M $CH_3COONH_4$ (Merck, A1542) pH 4.0
- Glycogen (Thermo Fisher Scientific, R0551)
- EtOH 80% (-20°C), EtOH (Roth, 5054.3) 99% (-80°C)
- Formamide (Roth, 6749.3)
- TBE 10x (Roth, 3061.2)
- Rotiphorese® DNA-Sequencing system (Roth, A431.1)
- GelRed (Biotium, #41001)
- $H_2O$ milliQ
- PCR tubes (Biozym, 711080)
- 1.5 mL tubes (Roth, 7080.1)
- NanoSep filters (Pall, ODM45C34)

Template: RNA/synthetic oligo MH 898 - RNA oligo with $m^6A$ (SEQ ID NO: 1; IBA Lifesciences, Göttingen, Germany)

Reaction:

| Deamination reaction | | | |
|---|---|---|---|
| | Stock conc. | Vol [μL] | Final conc. |
| RNA | 2.5 μg/μl | 2.0 | |
| $CH_3COONa$/$CH_3COOH$ (diff. pH) | 3 M | 33.4 | 1 M |

(continued)

| Deamination reaction | | | |
|---|---|---|---|
| | Stock conc. | Vol [$\mu$L] | Final conc. |
| NaNO$_2$ | 5 M | 20.0 | 1 M |
| H$_2$O | | 44.6 | |
| | Total volume | 100.0 | |

Mix in PCR tubes on ice in following order:
H$_2$O, CH$_3$COONa/CH$_3$COOH, RNA, NaNO$_2$ → Vortex and centrifuge
Incubate @ 50, 60 & 70°C for 1, 3, 5, 10, 20, 30 min with peqSTAR 2X Thermocycler
(Settings: Heat lid to 110°C, Temp. 50, 60 or 70°C for 1, 3, 5, 10, 20, 30 min, Store at 4.0°C).
Immediately go to step Stop.

Stop:

Transfer deaminated solution into 1.5 mL tubes.

| EtOH precipitation | | | |
|---|---|---|---|
| | Stock conc. | Vol [$\mu$L] | Final conc. |
| Deaminated solution | | 100.0 | |
| CH$_3$COONH$_4$ | 5 M | 25.0 | 0.5 M |
| Glycogen | | 1.0 | |
| H$_2$O | | 124.0 | |
| | Total volume | 250.0 | |

Mix deaminated solution with CH$_3$COONH$_4$, glycogen and H$_2$O. Add 750 $\mu$L EtOH (-80°C), vortex and precipitate at -80°C for 1 h.
Centrifuge with Sigma 1-15 PK centrifuge (Settings: -5°C, 13.000 rpm, 15.493 x g) for 45 min.
Remove supernatant from tubes and wash pellets with 100 $\mu$L EtOH 80% (-20°C), centrifuge with Sigma 1-15 PK centrifuge (Settings: -5°C, 13.000 rpm, 15.493 x g) for 15 min.
Remove supernatant from tubes and let pellets air dry.

Purification:

a) Purification of small RNA (e.g. synthetic oligos, tRNA) for LC-MS measurements and Sequencing

Resuspend with 20 $\mu$L (10 $\mu$L H$_2$O & 10 $\mu$L Loading Buffer (90% Formamide, 10% TBE 10x)) and load the samples on a 10% denat. PAGE gel with an Amersham pharmacia biotech Electrophoresis power supply EPS 3501 XL (Settings: 10 W, 45 min), a CBS Scientific LSG-400-20 gel chamber and a Tristar VE5923 fan.
Stain gel with 1:3 GelRed for 10 min.
Scan gel with Amersham Biosciences Typhoon Trio+ Variable Mode Imager (Settings: Fluorescence, 200 microns, Platen, 1 Channel, Filter 610 BP 30, Laser Green 532 nm, Sensitivity normal, PMT 450 V).
Cut corresponding bands, smash gel pieces and collect in a new 1.5 mL tube.
Freeze gel pieces for 30 min. at -20°C and add 250 $\mu$L 0.5 M CH$_3$COONH$_4$.
Elute with Eppendorf Thermomixer comfort at 25°C and 750 rpm overnight.
Transfer everything in NanoSep filters and centrifuge with Sigma 1-15 PK centrifuge (Settings: 20°C, 10.444 rpm, 10.000 x g) for 10 min.
Transfer supernatant in a new 1.5 mL tube. Add 1 $\mu$L Glycogen, 750 $\mu$L EtOH (-80°C), vortex and precipitate at -80°C for 1 h.
Centrifuge with Sigma 1-15 PK centrifuge (Settings: -5°C, 13.000 rpm, 15.493 x g) for 45 min.
Remove supernatant from tubes and wash pellets with 100 $\mu$L EtOH 80% (-20°C), centrifuge with Sigma 1-15 PK centrifuge (Settings: -5°C, 13.000 rpm, 15.493 x g) for 15 min.

Remove supernatant from tubes and let pellets air dry.

Resuspend with 10 μL H₂O.

Measure concentration with NanoDrop 2000 Spectrophotometer (Settings: Nucleic Acid, Type RNA, Baseline correction 340 nm).

b) Purification of mRNA (fragments) for LC-MS measurements and Sequencing

Resuspend with 45 μL H₂O, add 1 μL glycogen, 5 μL 5 M CH₃COONH₄ and 150 μL EtOH (-80°C), vortex and precipitate at -80°C for 1 h.

Centrifuge with Sigma 1-15 PK centrifuge (Settings: -5°C, 13.000 rpm, 15.493 x g) for 45 min.

Remove supernatant from tubes and wash pellets with 100 μL EtOH 80% (-20°C), centrifuge with Sigma 1-15 PK centrifuge (Settings: -5°C, 13.000 rpm, 15.493 x g) for 15 min.

Remove supernatant from tubes and let pellets air dry.

Resuspend with 10 μL H₂O.

Measure concentration with NanoDrop 2000 Spectrophotometer (Settings: Nucleic Acid, Type RNA, Baseline correction 340 nm).

*Library preparation*

Materials:

- Antarctic phosphatase (NEB, M0289L)
- RNaseOUT (RNAse inhibitor) (Thermo Fisher Scientific, 10777019)
- 100 mM ATP (Thermo Fisher Scientific, PV3227)
- Polynucleotide kinase PNK (NEB, M0201L)
- EtOH 80% (-20°C), EtOH (Roth, 5054.3) 99% (-80°C)
- RNeasy MinElute Cleanup Kit (Qiagen, 74204)
- NEBNext® Multiplex Small RNA Library Prep Kit for Illumina® (Index Primers 1-48) (NEB, E7560S)
- GeneJET PCR Purification Kit (Thermo Fisher Scientific, K0702)
- H₂O milliQ
- PCR tubes (Biozym, 711080)
- 1.5 mL tubes (Roth, 7080.1)
- 1.5 mL Eppendorf® LoBind microcentrifuge tubes (Sigma Aldrich, Z666548)

1. Dephosphorytation of (fragmented) deaminated RNA

Mix the following components in a PCR tube:

| | |
|---|---|
| ◦ (Fragmented) deaminated RNA/water | 16 μL |
| ◦ 10X phosphatase buffer | 2 μL |
| ◦ Antarctic phosphatase (NEB) | 1 μL |
| ◦ RNaseOUT (RNAse inhibitor) | 1 μL |
| | 20 μL |

Incubate with peqSTAR 2X Thermocycler with the following settings:

Heat lid to 110°C.

37°C for 30 min.

65°C for 5 min.

store at 4°C.

2. Phosphorylation of fragmented RNA

Add the following components the PCR tube from step 1:

| | |
|---|---|
| ∘ $H_2O$ | 21.5 μL |
| ∘ 10X PNK buffer | 5 μL |
| ∘ 100 mM ATP | 0.5 μL |
| ∘ RNaseOUT (RNAse inhibitor) | 1 μL |
| ∘ PNK (Polynucleotide kinase NEB) | 2 μL |
| | 50 μL |

Incubate with peqSTAR 2X Thermocycler at 37°C for 60 min (heat lid to 110°C), then store at 4°C.

3. Purification of fragmented & repaired RNA (RNeasy MinElute Cleanup Kit, Qiagen)

∘ Transfer the sample to a new 1.5 mL tube and add 50 μL $H_2O$ to adjust the volume to 100 μL.
∘ Add 350 μL of RLT buffer and mix well.
∘ Add 675 μL of EtOH 99% and mix well by inversion.

Immediately proceed to the next step.

∘ Transfer a part (about 700 μL) of the sample to an RNeasy MinElute spin column (4°C, stored in the fridge).
∘ Centrifuge with Sigma 1-15 PK centrifuge (Settings: 25°C, 8.452 rpm, 8.000 x g) for 15 seconds.

Repeat the same with the rest of the sample.

∘ Transfer column to new tube. Add 500 μL of RPE buffer.
∘ Centrifuge with Sigma 1-15 PK centrifuge (Settings: 25°C, 8.452 rpm, 8.000 x g) for 15 seconds. Discard the flow-through.
∘ Add 750 μL of EtOH 80%.
∘ Centrifuge with Sigma 1-15 PK centrifuge (Settings: 25°C, 8.452 rpm, 8.000 x g) for 2 min.
∘ Place the column in a new 2 mL collection tube with the lid open and centrifuge with Sigma 1-15 PK centrifuge (Settings: 25°C, 13.000 rpm, 15.493 x g) for 5 min.
∘ Transfer the column to a new 1.5 mL tube. Add 10 μL $H_2O$ in the center of the column and let it stand for 1 min. and centrifuge with Sigma 1-15 PK centrifuge (Settings: 25°C, 13.000 rpm, 15.493 x g) for 1 min to elute. The recovered volume should be about 9 μL.

4. Ligation of the 3' SR Adaptor (NEBNext® Multiplex Small RNA Library Prep Kit)

Mix the following components in a PCR tube:

| | |
|---|---|
| ∘ Dephosphorylated RNA (about 100 ng) | x μL |
| ∘ 3' SR Adaptor for Illumina | 1.0 μL |
| ∘ $H_2O$ | ad 7.0 μL |

Incubate with peqSTAR 2X Thermocycler with the following settings:

Heat lid to 110°C.

70°C for 2 min.

Store at 4°C.

Transfer tube to ice.

Add the following components to the PCR tube:

| | |
|---|---|
| ∘ 3' Ligation Reaction Buffer (2X) | 10 μL |
| ∘ 3' Ligation Enzyme Mix | 3 μL |
| | 20 μL total |

Incubate with peqSTAR 2X Thermocycler with the following settings:

Heat lid to 110°C.

25°C for 1 h.

Store at 4°C.

5. Hybridize the Reverse Transcription Primer

Add the following components to the ligation mixture from the previous step and mix well:

| | |
|---|---|
| ∘ H$_2$O | 4.5 μL |
| ∘ SR RT Primer for Illumina | 1.0 μL |
| | 25.5 μL total |

Incubate with peqSTAR 2X Thermocycler with the following settings:

Heat lid to 110°C.

75°C for 5 min.

37°C for 15 min.

25°C for 15 min.

Store at 4°C.

6. Ligate the 5' SR Adaptor

With 15 minutes remaining at the previous step, aliquot 1.1 x N (amount of samples) μL of the 5' SR Adaptor into a separate PCR tube.
Incubate the adaptor with peqSTAR 2X Thermocycler with following settings:

Heat lid to 110°C.

70°C for 2 min.

Store at 4°C.

Immediately place the tube on ice. Keep the tube on ice and use the denatured adaptor within 30 minutes of denaturation.
Add the following components to the mix from step 5 and mix well:

| | |
|---|---|
| ∘ 5' SR Adaptor for Illumina (denatured) | 1 μL |
| ∘ 5' Ligation Reaction Buffer (10x) | 1 μL |
| ∘ 5' Ligase Enzyme Mix | 2.5 μL |
| | 30 μL total |

Incubate with peqSTAR 2X Thermocycler with following settings:

Heat lid to 110°C.

25°C for 1 h.

Store at 4°C.

7. Perform Reverse Transcription

Mix the following components in a in a PCR tube:

| | |
|---|---|
| ∘ Adaptor Ligated RNA from Step 6 | 30 μL |
| ∘ First Strand Synthesis Reaction Buffer | 8 μL |
| ∘ Murine RNase Inhibitor | 1.0 μL |
| ∘ ProtoScript II Reverse Transcriptase | 1.0 μL |
| | 40 μL total |

Incubate with peqSTAR 2X Thermocycler with following settings:

Heat lid to 110°C.

50°C for 1 h.

Store at 4°C.

Immediately proceed to PCR amplification.

8. Perform PCR Amplification

Add the following components to the RT reaction mix from the previous step and mix well:

| | |
|---|---|
| ∘ LongAmp *Taq* 2X Master Mix | 50 μL |
| ∘ SR Primer for Illumina | 2.5 μL |
| ∘ Index (X) Primer | 2.5 μL |
| ∘ $H_2O$ | 5 μL |
| | 100 μL total |

Incubate with peqSTAR 2X Thermocycler with following settings:

| | |
|---|---|
| Heat lid to 110°C. | |
| 94°C for 30 sec | Initial Denaturation |
| 15 cycles: | |
| 94°C for 15 sec | Denaturation |
| 62°C for 30 sec | Annealing |
| 70°C for 15 sec | Extension |
| 70°C for 5 min | Final Extension |
| Store at 4°C. | |

9. Purification (GeneJET PCR Purification Kit)

Transfer PCR reaction mix into new 1.5 mL tube.
Add a 1:1 volume of Binding Buffer to the PCR mixture (100 μL of reaction mixture + 100 μL of Binding Buffer). Mix thoroughly.

Transfer the solution to the GeneJET purification column. Centrifuge with Sigma 1-15 PK centrifuge (Settings: 25°C, 10351 rpm, 12.000 x g) for 60 sec. Discard the flow-through.

Add 700 µL of Wash Buffer (diluted: 45 mL with 225 mL EtOH 99%) to the GeneJET purification column. Centrifuge with Sigma 1-15 PK centrifuge (Settings: 25°C, 10351 rpm, 12.000 x g) for 60 sec. Discard the flow-through and place the purification column back into the collection tube.

Centrifuge the empty GeneJET purification column with Sigma 1-15 PK centrifuge (Settings: 25°C, 10351 rpm, 12.000 x g) for an additional 1 min to completely remove any residual wash buffer.

Transfer the GeneJET purification column to a clean 1.5 mL tube (DNA low binding tube).

Add 20 µL of Elution Buffer to the center of the GeneJET purification column membrane and centrifuge with Sigma 1-15 PK centrifuge (Settings: 25°C, 10351 rpm, 12.000 x g) for 1 min.

Discard the GeneJET purification column and store the purified DNA at -20°C.

Measure concentration with NanoDrop 2000 Spectrophotometer (Settings: Nucleic Acid, Type DNA, Baseline correction 340 nm).

10. Quality check (optional)

   o Perform a DNA HS chip on an Agilent Bioanalyzer 2100 or perform a HS-D1000 ScreenTape on an Agilent 4200 TapeStation System for size control and purity check
   ◦ Perform a QuBit HS DNA assay for concentration measurements

*Sequencing*

Before sequencing, libraries were pooled using different index reads ensuring diversity of nucleotides for signals in both red and green color channels.

Indeed, Illumina HiSeq and MiSeq use a green channel to read G/T nucleotides and a red channel to read A/C nucleotides.

With each sequencing cycle at least one nucleotide for each color channel must be present to ensure proper sequencing. In addition, to ensure the sequence diversity, PhiX Control v3 library (Illumina ref # FC-110-3001) was added to libraries to get 3-5 % of final concentration.

Libraries were denatured with fresh 2 N NaOH and diluted to 10 pM final concentration to ensure proper clustering on the flow cell.

Once clustering was finished, the samples were sequenced on MiSeq (e.g. in a SR50 run) using appropriate Illumina reagents.

Primary analysis of the data by FastQC (quality score distribution and adapter contamination).

Demultiplexing after sequencing consists of identification of barcode sequences attributed to each sample during the PCR amplification and of separation of sequencing reads into individual FASTQ files by sample. The demultiplexing step can be performed either using the integrated MiSeq RTA software, or in posttreatment, using the bcl2fastq utility of Illumina (Version 1.8.4). Demultiplexing was performed with 0 mismatch allowed in the barcode sequence. The resulting FASTQ files were treated and inspected for quality, the presence of adapters and overrepresented sequences using the FastQC utility.

*Bioinformatics*

The bioinformatic pipeline developed for the detection of RNA modifications on deaminated samples starts on demultiplexed reads from high-throughput sequencing machine as inputs. This pipeline follows 4 important steps:

   1) Trimming.
   2) Getting reads files in FASTA format.
   3) Alignment.
   4) Data analysis.

The first and second step are done by respectively one UNIX script for each of them, whereas R scripts are used for the remaining steps. The major challenge of this bioinformatic pipeline is the alignment part since the deamination alters the nucleotide composition of the samples, and it should be efficient enough to be able to identify modified positions during the data analysis. The other steps use standard methods and parameters, but must be compatible with the alignment script written in R. Here the four major bioinformatics steps created for the present invention are describes in a detailed manner.

Trimming (First Unix Script)

The RNA samples are sequenced from a HiSeq or MiSeq Illumina sequencing machine and are demultiplexed as described above. Other sequencing technologies could be used. The Illumina sequencing is a suitable technology to provide a sufficient amount of data with an overall good quality in a relative short time compared to the other sequencing technologies. The demultiplexing step converts the data in a compressed FASTQ format (.fastq.gz), which are used as inputs for the trimming. These data are then trimmed to remove the adapters (= flanking sequences) used for the library preparation which are not useful anymore at this point of the method. A lot of free trimming software exists such as Cutadapt or Trimmomatic. Here, Trimmomatic (version 0.38) is used, since this software already includes Illumina adapter sequences with the following standard parameters:

- **Seed mismatches** : **2** (2 mismatches allowed in order to trim a potential adapter).
- **Palindrome clip threshold** : **30** (Score threshold used for the "palindrome" mode : alignment between paired-end reads).
- **Simple clip threshold** : **10** (Score threshold used for the standard mode : direct alignment between the adapter sequences and the reads).
- **LEADING** : **30** (Cut bases off the start of a read, if below a Q-score of 30).
- **TRAILING** : **30** (Cut bases off the end of a read, if below a Q-score of 30).
- **SLIDINGWINDOW : 4:15** (Use of a window of 4 nucleotides long which comes across the read. For each window, if the average Q-score is below 15, these nucleotides are removed).
- **MINLEN : 8** (Drops any reads with a length below 8 nucleotides).
- **AVGQUAL : 30** (Drops any reads which got an average Q-score below 30).

FASTA format (Second Unix Script)

Once the trimming is finished, the data are still in a compressed FASTQ format. In order to make them accessible for the R software, these are converted to an uncompressed FASTA format. There are only slight differences between FASTQ and FASTA formats:

- Names of the reads in FASTQ format begin with a '@', whereas they begin with a '>' in FASTA.
- FASTQ contains encoded Q-score for each nucleobase of every reads, which is not present anymore in FASTA format.

The conversion is done by text replacement, thanks to the UNIX command: "sed -n "1~4s/^@/>/p;2~4p" which replaces every beginning '@' by a '>' and gets rid of the encoded Q-score part. This UNIX script also includes some miscellaneous parts, like rename the data files or move them to a new folder for example.

Alignment (First R script)

Due to the particular deamination of the samples, almost all the nucleic bases are modified, resulting in a significant difference between the theoretical reference sequence and those obtained after sequencing. To successfully align the sequences obtained with the reference sequence, a specific pairing between the different bases must then be made. Conventional alignment software is not able to do such matching. Thus, a brand new alignment algorithm has been developed for the present invention. This alignment algorithm allows for control over the entire alignment process. The algorithm is written under the R software (version 3.4.4), using the "seqinr" packages (version 3.4-5) allowing the manipulation of nucleic sequences by R and the reading of FASTA files among others and "biostrings" (version 2.46.0) containing different nucleotide sequence alignment algorithms. Once the FASTA files of the samples are imported into R, each sequence is aligned with the reference sequence ("Pairwise alignment") using the Smith-Waterman alignment algorithm, allowing "local alignment" of the sequences. This alignment mode is used instead of Needleman-Wunsch's, also called "global alignment", because local alignment overrides some of the artifacts that may appear depending on the library preparation kit used; deamination made this task very challenging. If the trimming is incomplete (some partial adapter sequences remain in the data for example), aligning in "local" mode automatically excludes these artifacts. Local alignment follows a customized substitution matrix, thus imposing the matching rules that the program must follow.

The substitution matrix:
With partially deaminated samples, the bases can be modified in their deaminated complement or remain the same. In theory, this would give us two possible matches for each base.

Nevertheless, some exceptions remain:

- Uridine (as thymidine in complementary DNA) is not affected by deamination, so it can only be matched with itself.
- Guanosine is deaminated to xanthosine, but xanthosine binds to guanosine. So guanosine matches only with itself.

It is therefore necessary to allow the program to align the cytidines of the reference sequence with cytidines and thymidines of the samples, but at the same time not to allow a single type of matching with the thymidines of the reference: A thymidine-thymidine matching.

However, this poses a problem for the alignment algorithm, as it requires a symmetric substitution matrix to work. If the pairing of cytidines with thymidines is allowed, it must be done in both directions: from the reference to the sample (which is wanted), but also in the other direction (resulting in a pairing of thymidines with cytidines, which is not wanted). To remedy this, a temporary modification of the reference sequence is made:

- The thymidines, noted T in FASTA format, are all converted to the letter S.
- The guanosines, noted G in FASTA format, are all converted to the letter H.

This allows to write specific matching rules for these two nucleotides between the reference and the samples: While allowing the matching between the cytidines of the reference, which remains unchanged, and the thymidines of the samples, the replacement of the thymidines by the letter S in the reference avoids the matching of these bases with the cytidines present in the samples, thus giving the matching rules that are desired ("single-way pairing").

That is, the first time to our knowledge that we have heard this specific way of pairing. In fact, the mapper software available on internet do some standard pairing (T aligned with T, C aligned with C...) so they do not need to distinguish which sequence is the reference and which sequence comes from the sample. That is why the symmetrical substitution matrix exists. However, for our case, we need such distinction to drastically improve the alignment of the samples. The "single-way pairing" is a new idea to overcome such unusual base pairing using a symmetrical substitution matrix.

This results in the following matrix:

|   | A | T | G | C | S | H | N |
|---|---|---|---|---|---|---|---|
| A | 1 | -3 | 1 | -3 | -3 | -3 | 0.25 |
| T | -3 | 1 | -3 | 1 | 2 | -3 | 0.25 |
| G | 1 | -3 | 1 | -3 | -3 | 2 | 0.25 |
| C | -3 | 1 | -3 | 1 | -3 | -3 | 0.25 |
| S | -3 | 2 | -3 | -3 | 2 | -3 | 0.25 |
| H | -3 | -3 | 2 | -3 | -3 | 2 | 0.25 |
| N | 0.25 | 0.25 | 0.25 | 0.25 | 0.25 | 0.25 | 0.25 |

This matrix can be summarized as follows:

- If the match is correct, add +1 (for adenosine and cytidine) or +2 (for guanosine or thymidine) to the alignment score.
- If mismatch, decrease the alignment score by 3.

There is also penalties for formation of "gaps" (due to insertions/deletions of some nucleotides into the reads): the alignment score decreases by 5 if a gap is opened, and decreases for an additional 3 every time the gap is extended (same penalty as a mismatch).

The program thus created, taking into account the Smith-Waterman alignment algorithm and this matrix, will try to find for each sequence of samples the alignment with a maximum score. Then, depending on the size of the aligned sequences and the score value obtained, thresholds can be set up to exclude sample sequences too small to be aligned with certainty, or having an alignment score too low to be considered similar to the reference sequence.

Data analysis (Second R script)

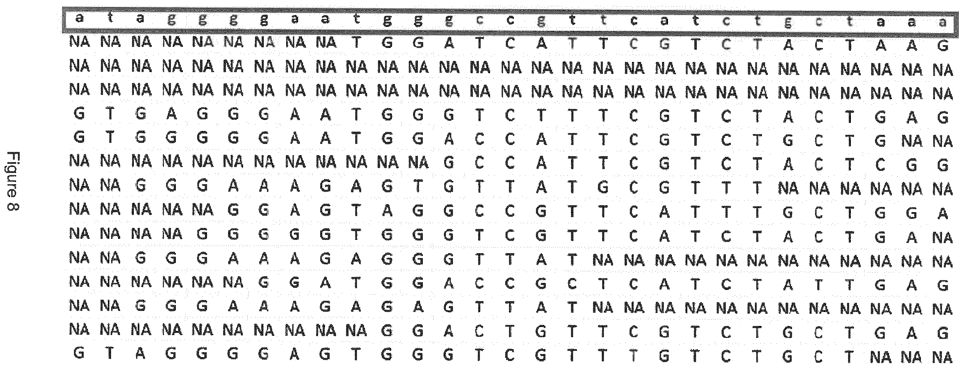

The output of the alignment script is an alignment matrix written in .csv format, which is structured as shown in Figure 8.

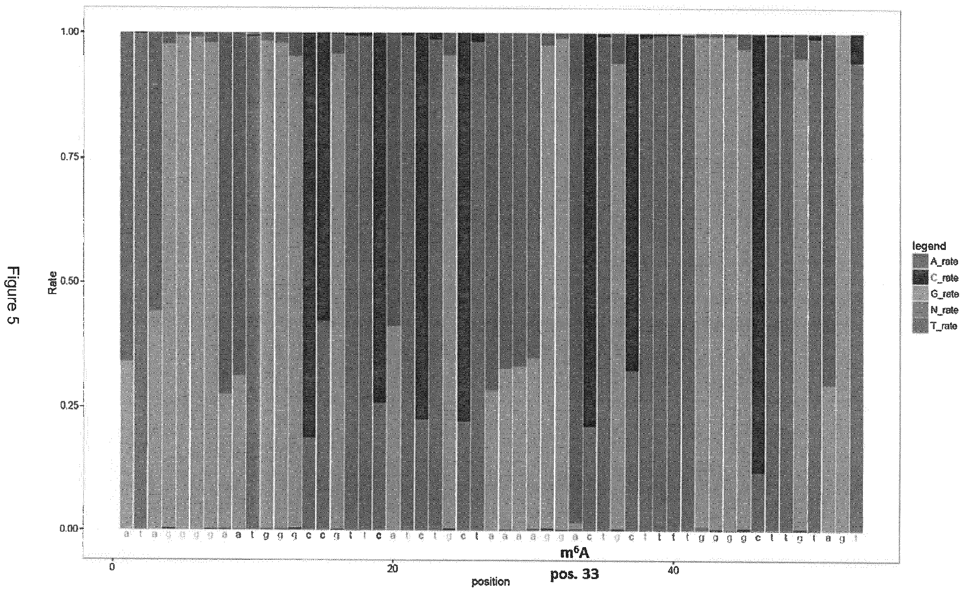

The first row of the matrix boxed in red and written in lower case corresponds to the (unmodified) reference sequence. All the remaining rows correspond to the aligned reads. From this matrix, the number and the rate of A, G, C or T aligned is calculated for every column (*i.e.,* for every bases of the reference). This allows establishing a graph as shown in Figure 5 to represent the overall alignment.

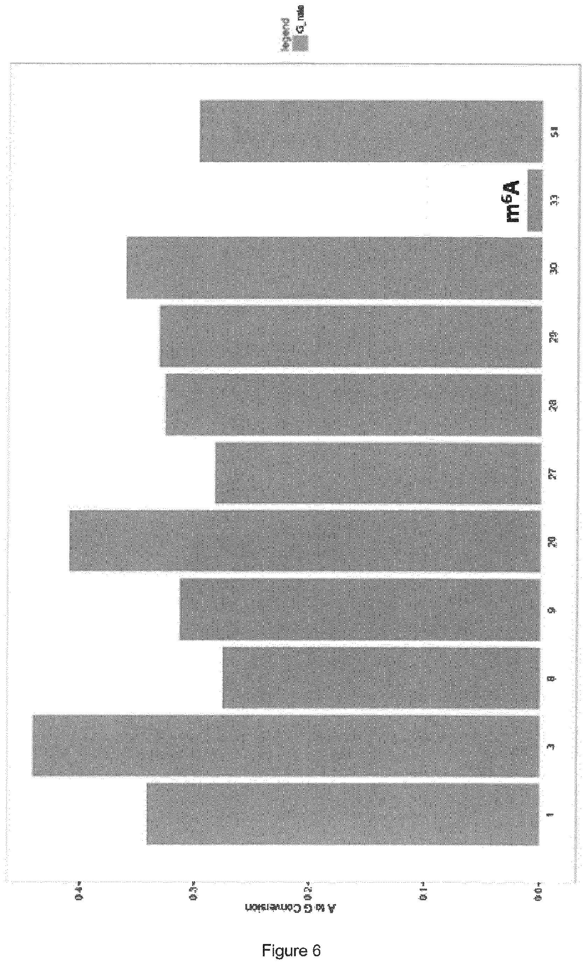

Then, all positions corresponding to an Adenosine according to the reference are extracted with their respective Adenosine alignment rate and Guanosine alignment rate. The positions where the Guanosine alignment rates are considerably lower than the others should be m6A, since this modification is not affected by the deamination process which leads to A/G conversion (Fig. 6).

Example 1:

RNA deamination

Ribonucleic acids react with nitrous acid. Under certain reaction conditions, this reaction leads to selective oxidative deamination of the primary amines of nucleobases and nucleic acids. The reactions of the primary amine-bearing adenosine, cytidine and guanosine have been reinvestigated. It could also be shown that m6A with its secondary amine is not deaminated, but shows a conversion to $N^6$-methyl-$N^6$-nitroso-adenosine.

In order to test the applicability of RNA deamination in Next-Generation-Sequencing approaches, treated RNA templates were tested by means of library preparation and subsequent sequencing. In these tests, the applicability of the deamination protocol to the subsequent library preparation and sequencing in particular proved to be very difficult. A sequencing of deaminated RNA samples with the conventional protocols was not possible.

Finally, the complex adaptation of the deamination and the subsequent enzymatic processing of the samples with accurately selected conditions surprisingly led to useful data in sequencing.

In the further processing, the bioinformatic analysis of the sequencing data turned out to be a special challenge. With conventional, open-source mapping tools, it was not possible to evaluate the data. It was necessary to devise the sophisticated algorithm of the present invention which finally allowed the processing and analysis of the data. This algorithm surprisingly delivered outstanding results.

All in all, every single step had its own difficulties and only individual troubleshooting as well as the successful connection and mutual adjustment of treatment, library preparation, sequencing and bioinformatics according to the present invention enabled the distinguishability between m6A and unmethylated adenosines as well as to unambiguously determine m6A positions in RNA templates.

Example 2:

Reaction kinetics

In order to observe the exact progress of the deamination reaction and to evaluate the reactivity of the nucleotides, tests on reactivity kinetics were performed. For this purpose, a synthetic m6A-bearing RNA template (5'-AUAG-GGGAAUGGGCCGUUCAUCUGCUAAAAGG(m6A)CUGCUUUUGGGG CUUGUAGU-3'; SEQ ID NO: 1) was treated with different reaction conditions, purified and then analyzed by LC-MS analytics. Base conversion could be achieved in a well-defined, predictive and programmable manner: during the chemical treatment, the primary amine-bearing nucleotides adenosine (A), cytidine (C) and guanosine (G) are converted into inosine (I), uridine (U) and xanthosine (X) (Fig. 2). Uridines in the RNA template remain unaffected and m6A shows the reaction to $N^6$-methyl-$N^6$-nitroso-adenosine, but no deamination due to the secondary amine. Furthermore, possible disturbing side-products of adenosine, cytidine and guanosine through the treatment could be excluded by analyzing the LC-MS chromatograms in detail.

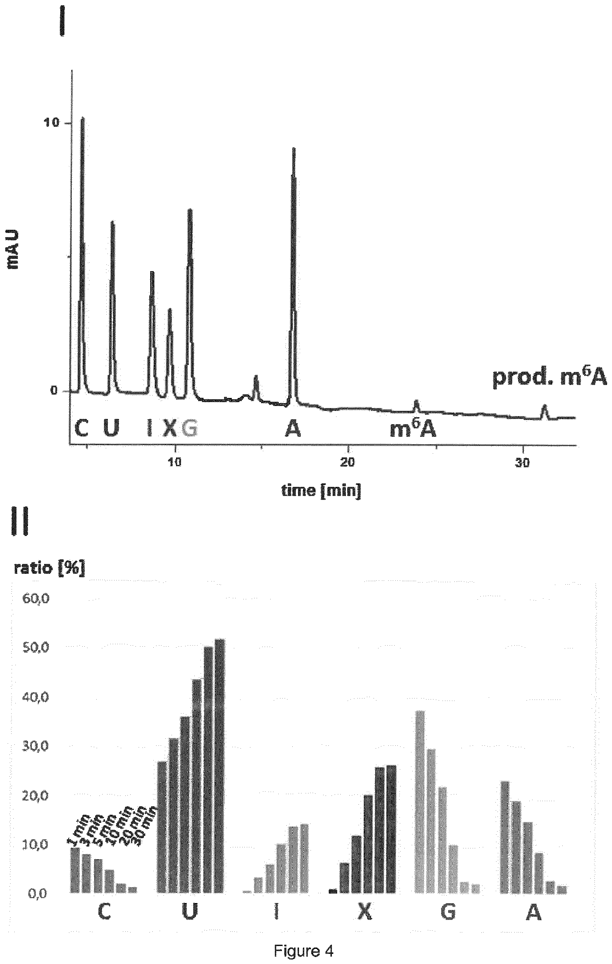

By screening different conditions, a correlation between reaction conditions and conversion levels could be found. This enabled close monitoring of the conversion of RNA nucleotides during treatment, prediction of the progression of the reaction and obtaining of the desired RNA with any required deamination level (Fig. 4). Additional deamination and LC-MS studies with an $N^6$-methyl-2'-deoxyadenosine (m6dA)-bearing DNA oligo confirm a potential application to DNA samples for the determination of m6dA.

Example 3:

Library preparation and sequencing

To check a possible sequencing application of the deamination treatment, the treated RNA template was used in library preparations. RNA templates with different deamination levels were selected to estimate the influence of base conversion on the enzymatic steps used during library preparation.

At the beginning, the preparation of samples that could be sequenced could not be achieved due to various problems during library preparation, in particular the reverse transcription of deaminated RNA into cDNA, which could not be achieved under the initial conditions. After testing countless different conditions and different levels of deamination, a library could be prepared for the first time and first sequencing data could be obtained, which however could not be evaluated qualitatively in an appropriate way. After trying out different library preparation protocols with moderate success, a suitable method could finally be found.

By combining adapter ligations at the 3' and 5' ends of the treated RNA, subsequent reverse transcription with a carefully selected reverse transcriptase, subsequent optimized amplification using PCR and specific Illumina sequencing primers, and corresponding size selection and purification of the samples, significantly improved and much more cost-efficient libraries could be obtained. Through some further adjustments, commercial kits for an optimized library preparation can also be used and thus samples can be processed in a fast and cost-effective manner.

It became clear that the reverse transcription of the RNA into cDNA plays the decisive role and essentially determines the successful differentiation between unmethylated adenosines and $m^6A$ in sequencing data. During this step, it must lead to an altered cDNA synthesis at adenosine positions due to the base conversion of unmethylated adenosines to inosines achieved during deamination pretreatment and the associated change in base-pairing behavior. In comparison to untreated, unmethylated adenosines and $m^6A$, which form a base pair with thymidine, inosine was expected to pair with cytidine, which is likewise true and supported by the data. In sequencing data, this change in base-pairing after chemical treatment of the RNA template can be used to distinguish between unmethylated adenosines and $m^6A$. At $m^6A$ sites, adenosines can be detected through the unchanged base-pairing in the data sets, while guanosines are detected for the converted, unmethylated adenosines (Fig. 3). By mapping the data sets against the reference sequence, the $m^6A$ position becomes clearly visible and detectable (Figs. 5 and 6).

Example 4:

Bioinformatics

The bioinformatic evaluation of the sequencing data after prior deamination treatment and the associated base conversion is one of the major challenges of the present invention. Especially in the case of partial deamination, *i.e.,* a reaction in which nucleotides are randomly and incompletely converted, the mapping of the data sets against the reference sequence is problematic.

In order to enable a complete and undisturbed analysis, a mapping tool must be used which can use the IUPAC ambiguity codes, for example to allow mapping of A and G at treated, unmethylated adenosine sites. Since most open-source mapping tools do not or only incompletely integrate this feature, a sophisticated mapping code based on the Smith-Waterman algorithm and the programming language R has been developed to circumvent these limitations by applying local sequence alignment with a specific custom substitution matrix. This enabled easy adaptation of the mapping to the requirements of the present invention.

Example 5:

Scoring

In order to find clear-cut criteria for assigning $m^6A$ or $m^4C$ to a given position in an RNA analyzed according to the present invention, two different scoring systems were established and evaluated.

To this end, samples of the $m^6A$-bearing RNA according to SEQ ID NO: 1 having increasing concentrations of a respective RNA without $m^6A$ (5'-AUAGGGGAAUGGGCCGUUCAUCUGCUAAAAGG**A**CUGCUUUUGGGGCUU GUAGU-3'; SEQ ID NO: 2) were prepared (100, 90, 80, 70, 60, 50, 40, 30, 20, and 10% $m^6A$-bearing RNA (SEQ ID NO: 1) with the remainder being the RNA according to SEQ ID NO: 2), and subjected to the method of the present invention, including or not including a step of enriching the $m^6A$-containing RNAs by immunoprecipitation in the sample prior to subjecting the same to deamination. Detection of $m^6A$ in position 33 of SEQ ID NO: 1/2 was considered to be confirmed using two different significance criteria, the first being a signal-to-noise ratio (S/N-ratio) of 3 or higher, as determined by the formula

$$S/N\text{-}ratio_i = \frac{average(R_{A>G})}{R_{A>G}(i)}$$

wherein

*S/N-ratio$_i$* is the signal-to-noise ratio in a given position i, *i.e.,* position 33 in this experiment,
*average(R$_{A>G}$)* is the average A-to-G-conversion ratio in the other positions of the test sequence, and
*R$_{A>G}$(i)* is the A-to-G-conversion ratio in position i.

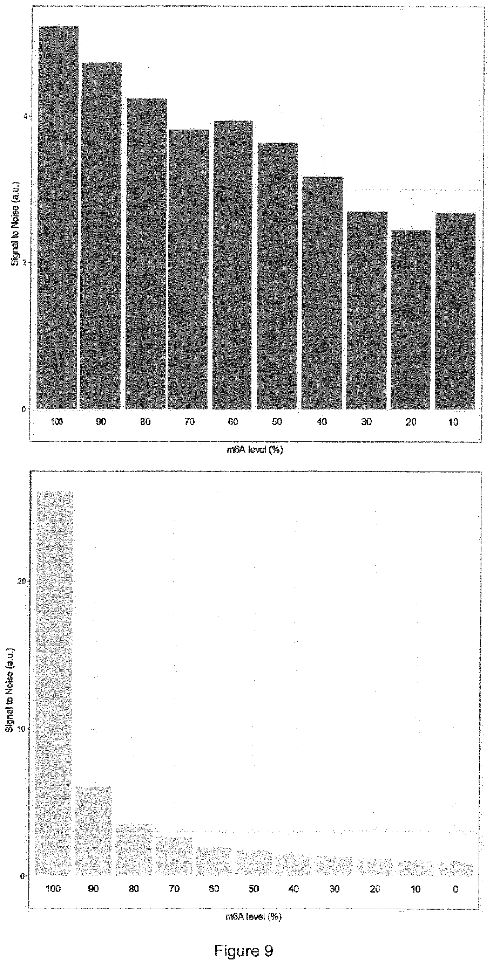

As can be taken from Figure 9, m$^6$A could be detected according to this criteria in the samples without immunoprecipitation down to an m$^6$A content in the sample of 80%, and in the sample with immunoprecipitation down to an m$^6$A content in the sample of 40%.

As the second criteria, the p-value as determined in a Student's t-test comparing the A-to-G-conversion ratio in position 33 of SEQ ID NO: 1/2 to the A-to-G-conversion ratios in the other positions of the test sequence being below 0.05 was used. A p-value of 0.05 corresponds to a 5% or lower probability of the A-toG-conversion ratio in position 33 of SEQ ID NO: 1/2 not being different from the A-to-G-conversion ratios in the other positions of the test sequence. Said p-value corresponds to a p-value score of 1.3 or higher, as determined by the formula

$$p\text{-}value\ score = -log_{10}(p\text{-}value).$$

As can be taken from Figure 10, m$^6$A could be detected according to this criteria in the samples without immunoprecipitation down to an m$^6$A content in the sample of only 20%, and in the sample with immunoprecipitation down to an m$^6$A content in the sample of as low as 10%.

**Claims**

1. A method for detecting the position of a nucleotide modification, selected from the group consisting of $N^6$-methyl-adenosine (m$^6$A) and $N^4$-methylcytidine (m$^4$C) in one or more RNA species, comprising the steps:

   (a) providing a sample comprising said one or more RNA species,
   (b) subjecting said sample to a deamination reaction to obtain deaminated RNAs,
   (c) subjecting said deaminated RNAs to reverse transcription using a reverse transcriptase (RT) to obtain respective cDNAs,
   (d) sequencing said cDNAs, and
   (e) determining the presence of m$^6$A at a given position in the RNA, in case the sequencing data show an adenosine (A) in the corresponding position, or the presence of m$^4$C at a given position in the RNA, in case the sequencing data show a cytidine (C) in the corresponding position.

2. The method of claim 1, wherein the deamination reaction is performed in a manner to result in completely deaminated RNAs.

3. The method of claim 1, wherein the deamination reaction is performed in a manner to result in partially deaminated RNAs, and wherein
   step (e) comprises the steps

   (e1) aligning the sequences determined in step (d) (test sequences) with respective reference sequences,
   (e2) determining for each A in a reference sequence the number of test sequences showing a dA in the respective position (n(A)) and the number of test sequences showing a dG in the respective position (n(G)), and/or determining for each C in a reference sequence the number of test sequences showing a dC in the respective position (n(C)) and the number of test sequences showing a dT in the respective position (n(T)),
   (e3) determining for each of the respective positions an A-to-G-conversion ratio and/or a C-to-T-conversion ratio, and
   (e4) determining

   (i) the presence of m$^6$A at a given position in the RNA, in case the respective A-to-G-conversion ratio is significantly lower compared to the A-to-G-conversion ratios in other positions of the test sequence, or
   (ii) the presence of m$^4$C at a given position in the RNA, in case the C-to-T-conversion ratio is significantly lower compared to the C-to-T-conversion ratios in other positions of the test sequence.

4. The method of claim 3, wherein the reference sequences are

   (i) derived from a database, or
   (ii) are generated in the method of claim 3, wherein

   in step (b), a first aliquot of said sample is subjected to said deamination reaction, whereas a second aliquot of said sample is left untreated as a reference,
   in step (c), said deaminated RNAs and the untreated reference RNAs are subjected to reverse transcription using a reverse transcriptase to obtain respective cDNAs and reference cDNAs, and
   in step (d), sequencing of said cDNAs and reference cDNAs yields sequencing data for the cDNAs (test sequences) and reference cDNAs (reference sequences).

5. The method according to claim 3 or claim 4, wherein in step (e1), the alignment of the test sequences with the reference sequences uses the following matching rules:

   (i) for 2'-deoxyribothymidine (dT) sites in the reference sequence, the matching of dT in the test sequence is allowed,
   (ii) for 2'-deoxyriboguanosine (dG) sites in the reference sequence, the matching of dG in the test sequence is allowed,
   (iii) for 2'-deoxyriboadenosine (dA) sites in the reference sequence, the matching of dA and dG in the test sequence is allowed, and
   (iv) for 2'-deoxyribocytidine (dC) sites in the reference sequence, the matching of dC and dT in the test sequence is allowed.

6. The method according to any one of claims 3 to 5, wherein in step (e3), the A-to-G-conversion ratio and/or a C-to-T-conversion ratio for each position is determined as

$$R_{A>G} = \frac{n(G)}{n(A) + n(G)}$$

and

$$R_{C>T} = \frac{n(T)}{n(C) + n(T)}$$

wherein

$R_{A>G}$ is the A-to-G-conversion ratio,
$R_{C>T}$ is the C-to-T-conversion ratio,
$n(G)$ is the number of test sequences showing a dG in said position,
$n(A)$ is the number of test sequences showing an dA in said position,
$n(T)$ is the number of test sequences showing a dT in said position, and
$n(C)$ is the number of test sequences showing a dC in said position.

7. The method of any one of claims 3 to 6, wherein the A-to-G-conversion ratio or the C-to-T-conversion ratio in a given position is considered to be significantly lower compared to the A-to-G-conversion ratios or the C-to-T-conversion ratios in other positions of the test sequence, in case

(i) the signal-to-noise ratio (S/N-ratio) is above a default value, wherein

$$S/N\text{-}ratio_i = \frac{average(R_{A>G})}{R_{A>G}(i)}$$

or

$$S/N\text{-}ratio_i = \frac{average(R_{C>T})}{R_{C>T}(i)}$$

wherein

$S/N\text{-}ratio_i$ is the signal-to-noise ratio in a given position i, $average(R_{A>G})$ is the average A-to-G-conversion ratio in the other positions of the test sequence,
$average(R_{C>T})$ is the average C-to-T-conversion ratio in the other positions of the test sequence,
$R_{A>G}(i)$ is the A-to-G-conversion ratio in position i, and
$R_{C>T}(i)$ is the C-to-T-conversion ratio in position i; and/or

(ii) the p-value as determined in a Student's t-test comparing the A-to-G-conversion ratio or the C-to-T-conversion ratio in a given position to the A-to-G-conversion ratios or the C-to-T-conversion ratios in other positions of the test sequence is below a default value.

8. The method according to any one of claims 1 to 7, wherein the deamination reaction is an oxidative deamination reaction.

9. The method according to any one of claims 1 to 8, wherein the deamination reaction is a chemical or an enzymatic deamination reaction.

10. The method according to any one of claims 3 to 9, wherein the partial deamination reaction is performed

(i) at a pH of 3 to 5, and
(ii) at a temperature of 40°C to 60°C, and
(iii) for a duration of 1 to 30 minutes.

11. The method according to any one of claims 1 to 10, further comprising

(i) a step of enriching m$^6$A- and/or m$^4$C-containing RNAs by immunoprecipitation in the sample provided in step (a) prior to step (b), and/or
(ii) a step of amplifying the cDNAs obtained in step (c) prior to sequencing in step (d).

12. The method according to any one of claims 1 to 11, wherein the sequencing in step (d) is performed using a high throughput sequencing (Next-Generation-Sequencing) method.

13. The method according to any one of claims 3 to 12, wherein the alignment in step (e1) is based on the Smith-Waterman- and/or the Needleman-Wunsch algorithm.

14. The method according to any one of claims 1 to 13, wherein the nucleotide modification is m$^6$A.

15. The method according to any one of claims 1 to 14, wherein said one or more RNA species encompass the entire transcriptome of a cell or tissue sample of interest.

Figure 1

Figure 2

Figure 3

Figure 4

Figure 5

Figure 6

Figure 7

Figure 8

Figure 9

Figure 10

Europäisches
Patentamt

European
Patent Office

Office européen
des brevets

## EUROPEAN SEARCH REPORT

Application Number

EP 19 17 0245

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/245128 A1 (HE CHUAN [US] ET AL) 30 August 2018 (2018-08-30) * paragraph [0145]; claim 1 * | 1,2,8,9, 11-15 | INV. C12Q1/6858 |
| X | MARK HELM ET AL: "Detecting RNA modifications in the epitranscriptome: predict and validate", NATURE REVIEWS GENETICS, vol. 18, no. 5, 20 February 2017 (2017-02-20), pages 275-291, XP055477162, GB ISSN: 1471-0056, DOI: 10.1038/nrg.2016.169 * page 277, left-hand column * | 1,2,8,9, 11-15 | |
| A | US 2017/044619 A1 (ROSE REBECCA [US] ET AL) 16 February 2017 (2017-02-16) * paragraph [0022]; claim 10 * | 1 | |

TECHNICAL FIELDS
SEARCHED      (IPC)

C12Q

The present search report has been drawn up for all claims

| Place of search | Date of completion of the search | Examiner |
|---|---|---|
| The Hague | 15 October 2019 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 0245

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2019

| Patent document cited in search report | Publication date | Patent family member(s) | Publication date |
|---|---|---|---|
| US 2018245128 A1 | 30-08-2018 | US 2018245128 A1<br>WO 2017040477 A1 | 30-08-2018<br>09-03-2017 |
| US 2017044619 A1 | 16-02-2017 | NONE | |

EPO FORM P0459

For more details about this annex : see Official Journal of the European Patent Office, No. 12/82

```
                        SEQUENCE LISTING

     <110>  Helm, Mark

     <120>  Detection of Nucleic Acid Modifications by Chemical Deamination

     <130>  H 3791EÙ - uh

     <160>  2

     <170>  PatentIn version 3.5

     <210>  1
     <211>  53
     <212>  RNA
     <213>  Artificial sequence

     <220>
     <223>  Synthetic m6A-containing RNA oligonucleotide


     <220>
     <221>  modified_base
     <222>  (33)..(33)
     <223>  m6a

     <400>  1
     auaggggaau gggccguuca ucugcuaaaa ggacugcuuu uggggcuugu agu          53


     <210>  2
     <211>  53
     <212>  RNA
     <213>  Artificial sequence

     <220>
     <223>  Synthetic RNA oligonuleotide

     <400>  2
     auaggggaau gggccguuca ucugcuaaaa ggacugcuuu uggggcuugu agu          53
```